# FASCICULE DE BREVET EUROPEEN

(11) **EP 3 091 960 B1**
(45) Date de publication et mention de la délivrance du brevet: **31.07.2024**
(21) Numéro de dépôt: 15705628.4
(22) Date de dépôt: 09.01.2015
(51) Int. Cl.: A61K 8/60, A61K 8/97, A61Q 19/08, A61Q 19/00, A61K 36/81, A61P 17/00, A23L 33/105, A61Q 19/02

(54) **EXTRAIT VÉGÉTAL COMPRENANT DES SUCROSE-ESTERS EN TANT QUE PRINCIPE ACTIF POUR SON UTILISATION DANS UNE COMPOSITION COSMÉTIQUE, DERMATOLOGIQUE OU NUTRACOSMÉTIQUE**
PFLANZENEXTRAKT, SUKROSEESTER ALS WIRKSTOFF ZUR VERWENDUNG IN EINER KOSMETISCHEN, DERMATOLOGISCHEN ODER NUTRIKOSMETISCHEN ZUSAMMENSETZUNG
PLANT EXTRACT COMPRISING SUCROSE ESTERS AS AN ACTIVE AGENT FOR USE IN A COSMETIC, DERMATOLOGICAL OR NUTRICOSMETIC COMPOSITION

(30) Priorité: 10.01.2014 FR 1400051
(43) Date de publication de la demande: 16.11.2016
(73) Titulaire: Cosmo International Ingredients, 06250 Mougins (FR)
(72) Inventeur: LAVILLE, Rémi, F-06340 Drap (FR); CICCHETTI, Esmeralda, F-06130 Grasse (FR); GARNIER, Sébastien, F-06650 Le Rouret (FR); DUROURE, Leslie, 53 résidence les bois murés, 0613 (FR); MANZONE, Gérard, F-06460 Saint Vallier de Thiey (FR); BORSOTTO, Jérémie, 06580 Pégomas (FR)
(74) Mandataire: Renard, Emmanuelle
(86) Numéro de dépôt international: PCT/FR2015/000011
(87) Numéro de publication internationale: WO 2015/104484

(56) Documents cités:
- FR-A1- 2 896 155
- FRANCO L A ET AL: "Actividad antinflamatoria de extractos y fracciones obtenidas de càlices de Physalis peruviana L", BIOMEDICA: REVISTA DEL INSTITUTO NACIONAL DE SALUD, XX, CO, vol. 27, 1 January 2007 (2007-01-01), pages 110 - 115, XP002495323, ISSN: 0120-4157
- E.M TALERO BARRIENTOS ET AL: "Anti-inflammatory effect of the ether extract and major fraction of Physalis peruviana calyces in acute TNBS-induced colitis", ABSTRACT OF THE 6TH EUROPEAN CONGRESS OF PHARMACOLOGY (EPHAR 2012), 2012, XP055151389, Retrieved from the Internet <URL:http://www.pa2online.org/abstracts/vol10issue3abst610p.pdf> [retrieved on 20141106]
- DESAI N B ET AL: "Sucrose esters-an interesting class of substances for cosmetics. Saccharoseester Eine für Kosmetika interessante Stoffklasse", PARFUMERIE UND KOSMETIK, HUETHIG, HEIDELBERG, DE, vol. 66, no. 10, 1 January 1985 (1985-01-01), pages 629 - 632, XP009093019, ISSN: 0031-1952
- "International Cosmetic Ingredient Dictionary and Handbook, fourteenth edition 2012", vol. 2, 2012, pages: 2414, XP002732191

## Description

La présente invention se situe dans le domaine cosmétique, dermatologique et nutracosmétique.

L'invention concerne les sucrose-esters obtenus d'un extrait végétal provenant du calice d'une des nombreuses plantes de la famille des *Solanaceae*, du genre *Physalis* comprenant principalement un ou plusieurs sucrose-esters moyennement polaires à apolaires ayant un nombre de carbone des groupements acyles de C1 à C10 pour leur utilisation dans le traitement dermatologique des pathologies impliquant une sécheresse pathologique de la peau, des muqueuses ou des phanères, pour dépigmenter la peau et pour favoriser la cicatrisationL'invention concerne encore une composition cosmétique ou nutracosmétique comprenant comme principe actif, dans un milieu physiologique adapté, ces sucrose-esters. Elle concerne encore le procédé de préparation d'un mélange de sucrose-esters dudit extrait végétal.

Les compositions cosmétique, dermatologique et nutracosmétique sont destinées à améliorer l'hydratation, la fonction barrière de l'épiderme, à favoriser la cicatrisation, à dépigmenter la peau et à lutter de manière préventive et/ou curative contre le vieillissement cutané.

La peau est un organe vital composé de plusieurs couches (derme, couches prolifératives et *stratum corneum*) qui recouvre toute la surface du corps et assure essentiellement une fonction de barrière vis-à-vis du milieu extérieur. Cette fonction barrière repose en particulier sur la qualité de l'épiderme qui dépend notamment de l'état du stratum corneum et de l'équilibre entre la prolifération et la différenciation des kératinocytes épidermiques.

Les kératinocytes sont des cellules constituant 90% de la couche superficielle de la peau (épiderme) et des phanères (ongles, cheveux, poils). Ils synthétisent la kératine, une protéine fibreuse et insoluble dans l'eau, qui assure à la peau sa propriété d'imperméabilité et de protection extérieure. L'épiderme est divisé en quatre couches basées sur la morphologie des kératinocytes, lesquels passent progressivement de la couche basale vers les couches supérieures par différenciation cellulaire jusqu'au *stratum corneum* ou ils forment une couche de cellules mortes nommées *squames,* par apoptose. Cette couche constitue une barrière de protection et réduit la perte d'eau de l'organisme. Les kératinocytes sont en perpétuel renouvellement. Ils mettent environ 1 mois pour aller de la couche basale au *stratum corneum* mais ce processus peut être accéléré en cas d'hyperprolifération de kératinocytes (psoriasis).

La modulation de la différenciation des kératinocytes est un processus biologique qui peut intervenir dans le traitement des manifestations du vieillissement cutané, que ce soit tant dans la fonction barrière de la peau que dans la formation des rides. Comme actif inhibant la différentiation des kératinocytes on peut citer les rétinoïdes (rétinol, acide rétinoïque entre autres) qui sont reconnus comme principes actifs cosmétiques et dermatologiques agissant contre de nombreux problèmes cutanés tels que le psoriasis, l'acné et les rides.^{21,22} Cependant, les rétinoïdes induisent une inflammation de la peau incitant des laboratoires de recherche a investiguer de nouveaux actifs rétinoïde-like inhibiteurs de la différenciation des kératinocytes non inflammatoires.²³

Les fibroblastes sont des cellules présentes dans le tissu conjonctif, elles sont parfois appelées cellules de soutien. Ce sont notamment des cellules résidentes du derme qui en assurent la cohérence et la souplesse. Elles sécrètent des protéoglycanes et des glycoprotéines. Elles sécrètent notamment du collagène, de l'élastine et des fibrillines dont la fibrilline 1.

La fibrilline 1 est une glycoprotéine qui appartient à une famille de protéines comprenant trois membres, dont leur régulation reste mal connue aujourd'hui. Elle est sécrétée à la fois par les fibroblastes et par les kératinocytes. Elle constitue l'élément majeur des microfibrilles dans les matrices extracellulaires élastiques et non élastiques. La fibrilline 1 participe donc à la formation des fibres et leurs propriétés fonctionnelles et structurales jouent un rôle primordial dans l'élasticité du tissu conjonctif, notamment au niveau de la jonction dermo-épidermique. En effet, elle permet un ancrage des cellules épidermiques aux fibres élastiques du derme.

Au niveau du derme, on observe avec l'âge une diminution d'activité des fibroblastes avec une baisse de leur prolifération, de biosynthèse et d'échanges avec la matrice extracellulaire, ainsi qu'une altération des fibres de soutien tant qualitative que quantitative.

Au niveau de la jonction dermo-épidermique apparaît un aplatissement qui entraîne une diminution de la cohésion entre ces deux zones (derme et épiderme), avec raréfaction des éléments du derme et ainsi que des fibres d'ancrage, ce qui a pour conséquence une baisse des échanges métaboliques et la formation des rides. Les vergetures résultent d'une distension trop rapide et trop intense de la peau qui a pour conséquence l'atteinte des cellules fibroblastiques associées à une altération prédominante du tissu collagénique et des glycoprotéines de structure. La formation des vergetures semble être une modification du métabolisme des fibroblastes entraînant alors une mauvaise cicatrisation, ainsi qu'une rupture de contact entre les cellules et la matrice tel que cité dans le brevet WO 2001007006²⁰.

La fibrilline 1 est citée, parfois avec l'élastine, comme protéine impliquée dans les processus de fermeté et d'élasticité^{9,10}, agissant dans le mécanisme anti-âge^{11, 12, 13, 14, 15}, et est notamment utilisée comme biomarqueur de l'accélération du vieillissement par les rayons ultraviolets^{16,17,18}, et/ou pour le traitement de certains processus cicatriciels comme pour les vergetures. L'activation de la synthèse de la fibrilline 1 permettrait ainsi de traiter les peaux vieillies intrinsèquement, dit génétiquement programmé ou chronologique, ou extrinsèquement, notamment dues à un stress oxydatif comme les UV, le tabac ou la pollution et de traiter les cicatrices de la peau.

Ainsi, les fibroblastes et les kératinocytes jouent un rôle primordial dans le processus biologique de la peau notamment par la sécrétion de la fibrilline 1, pour réguler la fermeté, l'élasticité, la cohésion cellulaire et dans le processus de cicatrisation et du vieillissement de la peau.

Au cours du vieillissement cutané, la peau devient sèche. Ainsi, il est très souvent constaté chez les sujets âgés, et notamment de plus de 50 ans, la manifestation d'une xérose ou d'une sécheresse des muqueuses, liée à une moindre sécrétion de sébum, à des modifications hormonales ou à des ralentissements du flux hydrique à travers l'épiderme. La peau est alors le siège de démangeaisons et de tiraillements, deux symptômes caractéristiques d'une peau sèche. Parmi les pathologies acquises se traduisant par une sécheresse de la peau, on peut citer la xérose induite par la photo-chimiothérapie et l'eczéma. Parmi les pathologies acquises provoquant une sécheresse de la bouche, ou xérostomie, on peut citer le syndrome de Sjogren ou la radiothérapie du cou. Enfin, parmi les pathologies impliquant une sécheresse des muqueuses, on peut citer les sécheresses oculaires ou vaginales.

Pour pallier à ces problèmes de sécheresse et vieillissement de la peau, il est communément utilisé des produits par voie topique destinés à restaurer la barrière cutanée. On peut citer les agents humectants, les agents filmogènes, les molécules de type rétinoïde et les agents capables de reconstruire la barrière cutanée comme le squalène par exemple.

La présente invention consiste principalement en la découverte qu'une certaine catégorie de sucrose-esters, présents dans certains extraits végétaux, ou synthétisés, présentent des activités biochimiques et biologiques intéressantes sur la peau, les phanères et les muqueuses notamment pour améliorer l'hydratation, la fonction barrière de l'épiderme et prévenir et/ou lutter contre les signes du vieillissement cutané.

Les sucroesters sont des acylesters d'un ou plusieurs groupement acyles avec un nombre de carbone plus ou moins important (C1 à C22 par exemple) et d'un sucre. Généralement, le sucre est le saccharose et on parle alors de sucrose-esters ou sucroesters. Les sucrose-esters possèdent un caractère amphiphile qui peut être modulé selon le degré d'estérification du sucre et la nature des groupements acyles.

Les sucrose-esters sont connus pour apporter une solution alternative aux industries du secteur des tensioactifs car ils présentent des avantages certains tels que leur innocuité, l'absence de goût et d'odeur et leur caractère non-ionique. Les applications des sucrose-esters en tant que tensioactifs sont diverses telles que dans le domaine de l'agroalimentaire, de la détergence, de la cosmétique et de la pharmaceutique.

Les sucrose-esters rencontrés sur le marché sont principalement d'origine synthétique et des voies de biosynthèse sont de plus en plus développées pour revendiquer un procédé éco-responsable et un produit 100% naturel.^{1,2} Les sucroesters naturels existent mais ils sont méconnus du secteur industriel. En effet, la famille des *Solanaceae,* dont les représentants les plus connus sont *Nicotiana tabacum* (le tabac) et *Solanum lycopersicum* (tomate), sont reconnus pour synthétiser et stocker des sucrose-esters dans leurs trichomes, moyens de défense contre de potentiels prédateurs.^{3,4}

Dans le cadre de recherche de sucrose-esters naturels, les laboratoires de recherche se sont interrogés sur l'utilisation de telles molécules en tant qu'actifs thérapeutiques. Les sucrose-esters possèdent des activités biologiques telles que anti-bactériens, anti-inflammatoires, antiparasitaires, et peuvent moduler la multi-résistance médicamenteuse.^{5,6} En comparaison avec les sucrose-esters synthétiques, les groupements acyles des sucrose-esters naturels peuvent aussi être de nature aromatique (benzoate, ...).⁷ Ces sucrose-esters aromatiques font l'objet de beaucoup de recherche en chimie thérapeutique comme agents antitumoraux.⁸

Le document EP 0 280 413 décrit l'utilisation de sucrose-esters pour augmenter la pénétration d'actifs biologiques à travers la peau. Ces sucrose-esters, obtenus par synthèse, sont le sucrose monolaurate ou alternativement le mélange de sucrose d'acides gras de noix de Coco dont l'acide gras majoritaire est l'acide laurique (C12).

Le document JP61271205 décrit que certains glucose-esters synthétiques à chaînes très longues (supérieures à 22 carbones), en combinaison avec le glycosylcéramide ou le céramide, sont utiles pour augmenter la mouillabilité, la flexibilité et l'élasticité de la peau.

Le document JP 2001 122731 décrit qu'un extrait de plante, sans indiquer la plante ou la partie de plante, est utile en cosmétique pour remédier à la sécheresse de la peau et en tant que détergent. Les solvants cités pour obtenir l'extraction sont essentiellement polaires et conduisent par conséquent à l'extraction de molécules polaires.

Le document JP 2011 051920 décrit l'utilisation d'un extrait de *Physalis peruviana* pour blanchir la peau et réduire les tâches de vieillesse. Dans ce document, c'est le fruit de *Physalis peruviana* qui est utilisé.

Le document de Franco L A et al. , « Actividad antiinflammatoria de extractos y fracciones obtenidas de calices de Physalis peruviana L », Biomedica, vol. 27, 1 Janvier 2007, divulgue un procédé d'extraction d'un extrait à partir du calice de *Physalis peruviana* pour envisager une activité anti-inflammatoire dudit extrait.

Le document de E.M Taleo Barrientos et al., « Anti-inflammatory effect of the ether extract and major fraction of Physalis peruviana calyces in acute TNBS-induced colitis », Abstract of the 6th European Congress of pharmacology, 2012*,* divulgue un procédé d'extraction d'un extrait à partir du calice de *Physalis peruviana* pour démontrer que deux nouveaux sucrose-esters présentent une activité anti-inflammatoire au niveau du colon.

Le document FR 2 896 155 divulgue l'utilisation d'extrait de Physalis, notamment alkenkegi, pour agir dans le domaine de la cosmétique contre le vieillissement. Le procédé d'extraction des molécules actives est une extraction hydrophile et polaire et les molécules extraites sont donc polaires, notamment les polyphénols, whitanolides et sucres.

Le document de Desai N B et al., « Sucrose esters - an interesting class of substances for cosmetics », Parfumerie und kosmetik, Huethig, Heidelberg, DE, vol.66, N°10, 1 janvier 1985*,* divulgue des esters de saccharose synthétiques qui sont intéressants pour les produits cosmétiques. Le tableau 1 décrit des sucrose-esters ayant un nombre de carbone des groupements acyle de C12 à C18. Ces sucrose-esters sont considérés comme hydratant, lissant et anti-irritant et permettent d'obtenir des produits démaquillants non-irritants pour la cosmétique et la pharmacie.

Outre leur propriété tensioactive et thérapeutique notamment anti-bactérienne, anti-inflammatoire et antiparasitaire, les sucrose-esters moyennement polaires à apolaires ayant un nombre de carbone des groupements acyles de C1 à C10 n'ont jamais été décrits comme possédant une activité cosmétique, dermatologique ou nutracosmétique au niveau de la peau, des muqueuses ou des phanères.

Il a en particulier été mis en évidence par les inventeurs que certains sucrose-esters moyennement polaire à apolaires provenant d'extrait végétal ont une activité biologique sur l'activation de la fibrilline 1 et sur l'inhibition de la différenciation des kératinocytes notamment. Lesdits sucrose-esters ont montré également de manière surprenante une activité biologique sur plusieurs cibles biologiques telles que le collagène I, III et IV, l'élastine, la fibrilline-1, l'involucrine, la mélanine et la fillagrine.

Ainsi, les inventeurs de la présente invention ont mis en évidence de manière surprenante un nouveau principe actif d'origine végétale capable d'activer plusieurs cibles biologiques liées au vieillissement, à l'hydratation et la dépigmentation. Les inventeurs ont notamment mis en évidence un nouveau principe actif d'origine végétale capable d'activer la synthèse de la fibrilline 1 par les fibroblastes et les kératinocytes et/ou d'inhiber la différenciation des kératinocytes et agir biologiquement sur plusieurs cibles biologiques telles que le collagène I, III et IV, l'élastine, la fibrilline-1, l'involucrine, la mélanine et la fillagrine, et ainsi améliorer l'aspect de la peau, des muqueuses et des phanères, de prévenir et/ou de lutter contre les manifestations du vieillissement cutané intrinsèque et/ou extrinsèque, d'aider le processus de cicatrisation notamment dans le cas des vergetures, de prévenir et ou de lutter contre la sécheresse de la peau, des muqueuses et/ou des phanères et de dépigmenter la peau.

Dans la présente invention, on entend par
✔ « quantité efficace de principe actif » la quantité nécessaire de molécules actives pour obtenir le résultat recherché, à savoir, permettre d'obtenir une activité biologique sur la peau, les phanères et/ou les muqueuses. On comprendra dans la présente invention « quantité efficace d'extrait végétal » ou « quantité efficace de sucrose-esters » sachant que l'extrait végétal selon l'invention comprend principalement des sucrose-esters comme principe actif.
✔ « principe actif » l'un ou plusieurs des sucrose-esters ou l'extrait végétal comprenant principalement l'un ou plusieurs des sucrose-esters.
✔ « application topique », le fait d'appliquer ou d'étaler le principe actif selon l'invention, ou une composition le contenant, à la surface de la peau, d'une muqueuse ou des phanères.
✔ « cosmétiquement ou dermatologiquement acceptable », que le principe actif selon l'invention, ou une composition le contenant, est approprié pour entrer en contact avec la peau ou une muqueuse sans provoquer de réactions de toxicité ou d'intolérance.
✔ « physiologiquement adapté ou acceptable » que les compositions conviennent à une utilisation topique ou transdermique, en contact avec les muqueuses, les phanères (ongles, cheveux, poils), le cuir chevelu et la peau de mammifère et plus particulièrement humaine, les compositions pouvant être ingérées ou injectées dans la peau, sans risque de toxicité, d'incompatibilité, d'instabilité, de réponse allergique.
✔ « milieu physiologiquement adapté ou acceptable » ou "excipient approprié" sans être limitatif, une solution aqueuse ou hydro alcoolique, une émulsion eau-dans-huile, une émulsion huile-dans-eau, une microémulsion, un gel aqueux, un gel anhydre, un sérum, une dispersion de vésicules, une poudre. Ce milieu physiologiquement acceptable forme ce que l'on appelle classiquement l'excipient de la composition.
✔ « biologiquement actif », « qui possède une activité *in vivo* ou *in vitro* caractéristique de l'activité du principe actif selon l'invention ».
✔ « manifestations cutanées du vieillissement », toutes modifications de l'aspect extérieur de la peau et des phanères dues au vieillissement comme, par exemple, les rides et ridules, la peau flétrie, la peau molle, la peau amincie, le manque d'élasticité et/ou de tonus de la peau, la peau terne et sans éclat ou les taches de pigmentation de la peau, la décoloration des cheveux ou les taches sur les ongles, mais également toute modification interne de la peau qui ne se traduit pas systématiquement par un aspect extérieur modifié comme, par exemple, toute dégradation interne de la peau consécutive à une exposition aux rayonnements ultraviolets (UV).
✔ "comprend principalement", le fait que les sucrose-esters obtenues par extraction d'une matrice végétale soient présents en majorité dans l'extrait végétal, c'est à dire à plus de 50% en poids, de préférence plus de 80%, de préférence encore plus de 90% jusqu'à 100%.
✔ « sucrose-esters synthétisés » toute manière d'obtenir des sucrose-esters, par hémisynthèse ou synthèse, que ce soit par synthèse chimique, biochimique ou biotechnologique.

L'invention consiste en des sucrose-esters obtenus d'un extrait végétal provenant du calice *de Physalis peruviana* comprenant principalement un ou plusieurs sucrose-esters moyennement polaires à apolaires ayant un nombre de carbone des groupements acyles de C1 à C10 pour leur utilisation dans le traitement dermatologique des pathologies impliquant une sécheresse pathologique de la peau, des muqueuses ou des phanères, pour dépigmenter la peau et pour favoriser la cicatrisation.

L'un ou plusieurs sucrose-esters moyennement polaires à apolaires ayant un nombre de carbone des groupements acyles de C1 à C10 est de formule générale suivante :

Les sucrose-esters moyennement polaires à apolaires ayant un nombre de carbone des 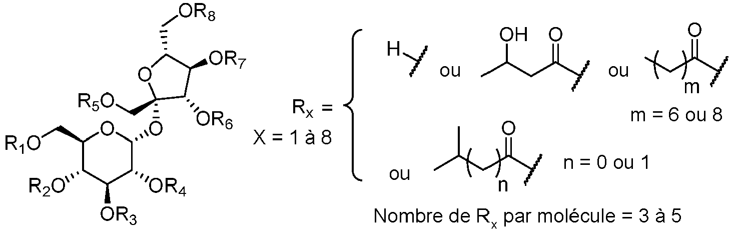 groupements acyles de C1 à C10 tel que décrit dans la formule générale sont présents dans l'extrait végétal à hauteur de 50 à 100% en poids de l'extrait total, de préférence de 80 à 100% en poids de l'extrait total et de préférence encore de 100% en poids de l'extrait total. Ces quantités sont basées sur les exemples 1, 2 et 3 qui ont permis de tester l'activité biologique des sucroses-esters présents dans l'extrait végétal selon l'invention.

Comme cela est indiqué dans les exemples ci-après, il est possible d'obtenir un extrait végétal comprenant de 80 à 100% de sucrose-esters moyennement polaires à apolaires ayant un nombre de carbone des groupements acyles de C1 à C10. Il est également possible de séparer les différents sucrose-esters obtenus pour obtenir un extrait purifié d'un seul sucrose-ester tel que décrit ci-dessus.

Un premier objet selon l'invention consiste en des sucrose-esters comprenant principalement un ou plusieurs sucrose-esters moyennement polaires à apolaires ayant un nombre de carbone des groupements acyles de C1 à C10 et obtenus d'un extrait végétal provenant du calice de *Physalis peruviana* ou sont synthétisés,les sucrose-esters étant choisis parmi :
- le 3-O-(2-methyl-1-oxopropyl)-β-D-fructofuranosyle(1→2)-3,4-bis(2-methylpropanoate)-2-decanoate-α-D-glucopyranoside,
- le 3-O-(3-methyl-1-oxobutyl)-(3-D-fructofuranosyle(1→2)-3,4-bis(2-methylpropanoate)-2-decanoate-α-D-glucopyranoside,
- le 3-O-(3-methyl-1-oxobutyl)-(3-D-fructofuranosyle(1→2)-3-(2-methylpropanoate)-2-decanoate-α-D-glucopyranoside,
- le 3-O-(2-methyl-1-oxopropyl)-β-D-fructofuranosyle(1→2)-3,6-bis(2-methylpropanoate)-2-decanoate-α-D-glucopyranoside,
- le 3-O-(2-methyl-1-oxopropyl)-β-D-fructofuranosyle(1→2)-3-(3-methylbutanoate)-2-decanoate-α-D-glucopyranoside.
- le 3-O-(2-methyl-1-oxopropyl)-β-D-fructofuranosyle(1→2)-3,4-bis(2-methylpropanoate)-2-nonanoate-α-D-glucopyranoside,
- le 3-O-(2-methyl-1-oxopropyl)-β-D-fructofuranosyle(1→2)-3,4-bis(2-methylpropanoate)-2-octanoate-α-D-glucopyranoside.
pour leur utilisation dans le traitement dermatologique des pathologies impliquant une sécheresse pathologique de la peau, des muqueuses ou des phanères, pour dépigmenter la peau et pour favoriser la cicatrisation.

Dans un mode de réalisation plus préférentiel encore selon l'invention, l'extrait végétal comprend un ou plusieurs des sucrose-esters suivants :
- le 3-O-(2-methyl-1-oxopropyl)-(3-D-fructofuranosyle(1→2)-3,4-bis(2-methyl pro-panoate)-2-decanoate-a-D-glucopyranoside,
- le 3-O-(3-methyl-1-oxobutyl)-(3-D-fructofuranosyle(1→2)-3,4-bis(2-methylpropa-noate)-2-decanoate-α-D-glucopyranoside.

Dans un mode de réalisation préférentiel, les sucrose-esters moyennement polaires à apolaires ayant un nombre de carbone des groupements acyles de C1 à C10 sont de formule générale suivante :

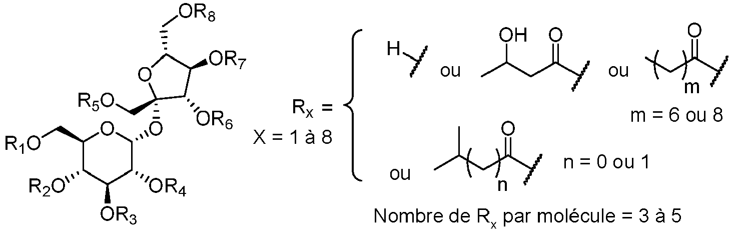

En effet, les sucrose-esters moyennement polaires à apolaires ayant un nombre de carbones des groupements acyles de C1 à C10, utiles en tant que principe actif dans des compositions cosmétique, dermatologique et nutracosmétique, peuvent provenir :
- soit du procédé d'extraction du calice d'une des nombreuses plantes de la familles des *Solanaceae*, du genre *Physalis,* permettant ainsi d'obtenir un extrait comprenant principalement un mélange de sucrose-esters moyennement polaires à apolaires ayant un nombre de carbones des groupements acyles de C1 à C10, ou après purification, une quantité d'un seul sucrose-ester moyennement polaires à apolaires ayant un nombre de carbones des groupements acyles de C1 à C10.
- soit de la synthèse d'un ou plusieurs des sucrose-esters moyennement polaires à apolaires ayant un nombre de carbones des groupements acyles de C1 à C10 tels que décrits. L'homme du métier étant à même de synthétiser de différentes manières connues les sucrose-esters individuellement pour constituer un principe actif à base de sucrose-esters selon l'invention.

Les sucrose-esters moyennement polaires à apolaires ayant un nombre de carbone des groupements acyles de C1 à C10 sont des acylesters d'un ou plusieurs groupements acyles avec un nombre de carbones de C1 à C10 et d'un saccharose.

Un second objet selon l'invention concerne une composition cosmétique comprenant comme principe actif, dans un milieu physiologique adapté, les sucrose-esters selon l'invention obtenus d'un extrait végétal provenant du calice de *Physalis peruviana* ou synthétisés.

La composition selon l'invention peut être formulée sous la forme de différentes préparations adaptées à une administration topique, à une administration orale, rectale, vaginale, nasale, auriculaire ou bronchique, à une administration parentérale.

Selon une première variante, les différentes préparations sont adaptées à l'administration topique et incluent les crèmes, les émulsions, les laits, les pommades, les lotions, les huiles, les solutions aqueuses ou hydro-alcooliques ou glycoliques, les poudres, les patchs, les sprays ou tout autre produit pour application externe.

Selon une deuxième variante, les différentes préparations sont adaptées à une administration orale ; l'extrait végétal comprenant les sucrose-esters pouvant entrer soit dans une composition alimentaire soit dans un complément alimentaire. Le complément alimentaire peut se présenter sous forme de gélules ou de capsules molles de gélatine ou végétales dans le cadre de la présente invention. Ledit complément alimentaire peut alors contenir de 0,01 à 100% en poids de l'extrait végétal.

Dans le cadre d'une utilisation cosmétique ou dermatologique, la composition sera avantageusement formulée sous la forme d'une préparation adaptée à une administration topique.

Dans le cadre d'une utilisation alimentaire, à visée nutritive ou cosmétique (cosmeto-food ou nutri-cosmétique ou nutracosmétique), la composition sera avantageusement formulée sous la forme d'une préparation adaptée à une administration orale. Elle pourra ne pas comprendre d'excipient et être constituée, en intégralité, de l'extrait végétal comprenant principalement les sucrose-esters ou des sucrose-esters synthétisés et correspondant avantageusement à la formule générale indiquée ci-avant.

Les compositions selon l'invention sont destinées plus particulièrement à une administration par voie topique. Ces compositions doivent donc contenir un milieu cosmétiquement et/ou dermatologiquement acceptable, c'est-à-dire compatible avec la peau et les phanères, et couvrent toutes les formes cosmétiques ou dermatologiques. Ces compositions pourront notamment être sous forme de crèmes, émulsions huile-dans-eau, ou eau-dans-huile ou émulsions multiples, solutions, suspensions, gels, laits, lotions, sticks ou encore de poudres, et adaptées à une application sur la peau, les lèvres et/ou les phanères. Ces compositions comprennent les excipients nécessaires à leur formulation, tels que solvants, épaississants, diluants, tensioactifs, anti-oxydants, colorants, conservateurs, parfums. Elles peuvent être utilisées comme produit de soin et/ou comme produit de maquillage de la peau.

La composition selon l'invention peut en particulier consister en une composition pour soins capillaires, et notamment un shampooing, un après-shampooing, une lotion traitante, une crème ou un gel coiffant, une lotion restructurante pour les cheveux, un masque, etc. La composition cosmétique selon l'invention peut être utilisée notamment dans les traitements mettant en oeuvre une application qui est suivie ou non d'un rinçage, ou encore sous forme de shampooing. La composition selon l'invention pourra avantageusement être utilisée dans les soins antipelliculaires. Elle peut également se présenter sous forme de teinture ou de mascara à appliquer au pinceau ou au peigne, en particulier sur les cils, les sourcils ou les cheveux.

Les compositions selon l'invention comprennent, en outre, tout additif communément utilisé dans le domaine d'application envisagé ainsi que les adjuvants nécessaires à leur formulation, tels que des solvants, des épaississants, des diluants, des anti-oxydants, des colorants, des filtres solaires, des agents auto-bronzants, des pigments, des charges, des conservateurs, des parfums, des absorbeurs d'odeur, des actifs cosmétiques ou pharmaceutiques, des huiles essentielles, des vitamines, des acides gras essentiels, des tensioactifs, des polymères filmogènes, etc.

L'INCI Dictionary & Handbook ("International Nomenclature of Cosmetic Ingrédients 13ème Ed. 2010) publié par "the Personal Care Products Council, Inc.", Washington, D.C.) décrit une grande variété, sans limitation, d'ingrédients cosmétiques et pharmaceutiques habituellement utilisés dans l'industrie du soin de la peau, qui conviennent pour être utilisés comme ingrédients additionnels dans les compositions selon la présente invention.

Des exemples non limitatifs de ces classes d'ingrédients additionnels incluent : les agents cicatrisants, les agents anti-âge, les agents anti-rides, les agents anti-atrophie, les agents hydratants, les agents adoucissants, les agents antibactériens, les agents antiparasitaires, les agents antifongiques, les agents fongicides, les agents fongistatiques, les agents bactéricides, les agents bactériostatiques, les agents antimicrobiens, les agents anti-inflammatoires, les agents anti-prurigineux, les agents anesthésiques, les agents antiviraux, les agents kératolytiques, les agents anti-radicaux libres, les agents anti-séborrhéiques, les agents anti-pelliculaires, les agents modulant la différenciation, la prolifération ou la pigmentation cutanée, les agents accélérateurs de pénétration, les agents desquamants, les agents stimulant ou inhibant la synthèse de mélanine, les agents blanchissants, dépigmentants, ou éclaircissants, les agents propigmentants, les agents auto bronzants, les agents inhibant la NO-synthase, les agents antioxydants, les agents piégeurs de radicaux libres et/ou anti-pollution atmosphérique, les agents anti-glycation, les agents raffermissants, les agents stimulant la synthèse des macromolécules dermiques ou épidermiques et/ou les agents capables de prévenir ou inhiber leur dégradation, les agents stimulant la synthèse de collagène, les agents stimulant la synthèse d'élastine, les agents stimulant la synthèse de décorine, les agents stimulant la synthèse de laminine, les agents stimulant la synthèse de défensine, les agents stimulant la synthèse de chaperon, les agents stimulant la synthèse de d'aquaporine, les agents stimulant la synthèse d'acide hyaluronique, les agents stimulant la synthèse de lipides et de composants du stratum corneum (céramides, acides gras, ...), les agents inhibant la dégradation du collagène, les agents inhibant la dégradation de l'élastine, les agents stimulant la prolifération des fibroblastes, les agents stimulant la prolifération des kératinocytes, les agents stimulant la prolifération des adipocytes, les agents stimulant la prolifération des mélanocytes, les agents stimulant la différenciation des kératinocytes, les agents stimulant la différenciation des adipocytes, les agents inhibant l'acétylcholinestérase, les agents stimulant la synthèse des glycosaminoglycanes, les agents réparant l'ADN, les agents protégeant l'ADN, les agents anti-démangeaison, les agents pour le traitement et/ou le soin de la peau sensible, les agents raffermissants, les agents anti-vergetures, les agents astringents, les agents régulant la production du sébum, les agents dermo-relaxants, les agents adjuvants de guérison, les agents stimulant la réépithélialisation, les agents adjuvants de réépithélialisation, les facteurs de croissance de cytokine, les agents calmants, les agents anti-inflammatoires, les agents agissant sur la circulation et/ ou microcirculation capillaire, les agents stimulant l'angiogénèse, les agents inhibant la perméabilité vasculaire, les agents agissant sur le métabolisme cellulaire, les agents destinés à améliorer la jonction dermo-épidermique, les agents induisant la pousse des cheveux et/ou des poils, les agents inhibant ou ralentissant la pousse des cheveux et/ou des poils, les agents myorelaxants, les agents anti-pollution et/ou anti-radicalaires, les agents stimulant la lipolyse, les agents amincissants, les agents anti-cellulite, les agents agissant sur la microcirculation, les agents agissant sur le métabolisme des cellules, les agents nettoyants, les agents de coiffage du cheveu, les stimulants de la pousse des cheveux, les écrans solaires, les écrans totaux, les agents de maquillage, les détergents, les produits pharmaceutiques, les agents émulsifiants, les émollients, les solvants organiques, les agents antiseptiques, les actifs déodorants, les milieux physiologiquement acceptables, les surfactants, les agents abrasifs, les absorbants, les composants esthétiques comme les parfums, les pigments, les teintures, colorants et colorants naturels, les huiles essentielles, les agents de toucher, les astringents cosmétiques, les agents anti-acné, les agents anti-coagulation, les agents anti-mousse, les antioxydants, les liguants, les additifs biologiques, les enzymes, les inhibiteurs enzymatiques, les inducteurs enzymatiques, les coenzymes, les agents chélatants, les extraits végétaux et dérivés de plantes, les huiles essentielles, les extraits marins, les agents venant d'un procédé de biofermentation et/ou de biotechnologie, les sels minéraux, les extraits cellulaires, les écrans solaires (les agents photoprotecteurs organiques ou minéraux qui sont actifs contre les rayons ultraviolets A et/ou B) les céramides, les peptides, les tampons, les agents de volume, les agents chélatants, les additifs chimiques, les colorants, les biocides cosmétiques, les dénaturants, les astringents médicinaux, les analgésiques externes, les agents filmogènes, comme les polymères, pour exacerber les propriétés filmogènes et la substantivité de la composition, les dérivés quaternaires, les agents augmentant la substantivité, les agents opacifiants, les ajusteurs et régulateurs de pH (ex. triethanolamine), les propellants, les agents réducteurs, les séquestrants, les agents décolorants et/ou éclaircissants de la peau, les agents conditionnant de la peau (i.e., humectants, incluant miscellanées et occlusifs), les substances retenant l'humidité, les alpha hydroxy acides, les béta hydroxy acides, les hydratants, les enzymes hydrolytiques épidermiques, les agents apaisants et/ou cicatrisants, les agents traitant la peau, les agents anti-rides, les agents capables de réduire ou traiter les poches sous les yeux, les agents exfoliants, les épaississants, les adoucissants, les polymères gélifiants, les vitamines et leurs dérivés, les agents mouillants, les agents pelants, les agents calmants, les agents curatifs de la peau, les lignanes, les conservateurs (i.e.phenoxyethanol et parabens), les anti-UV, les agents cytotoxiques, anti néoplasiques, agents modifiant la viscosité, les solvants non volatils, des agents perlants, les agents antitranspirants, les dépilatoires, vaccins, eau parfumée, agent restructurant la peau, les excipients, les charges, les minerais, les agents anti-mycobactériens, les agents anti-allergéniques, les antihistaminiques H1 ou H2, les anti-irritants, les agents stimulant le système immunitaire, les agents inhibant le système immunitaire, les agents répulsifs d'insectes, les lubrifiants, les pigmentants ou colorants, les agents hypopigmentants, les agents photo-stabilisants, et leurs mélanges, tant qu'ils sont physiquement et chimiquement compatibles avec les autres ingrédients de la composition et spécialement avec les actifs de la présente invention.

Par ailleurs, la nature de ces ingrédients additionnels ne doit pas altérer de manière inacceptable les bénéfices des actifs de l'invention. Ces ingrédients additionnels peuvent être synthétiques ou naturels comme par exemple les extraits de plantes ou venir d'un procédé de biofermentation. Des exemples additionnels peuvent être trouvés dans l'INCI Dictionary & Handbook

De tels ingrédients additionnels peuvent être choisis dans le groupe comprenant : les sucres aminés, glucosamine, D-glucosamine, N-acetyl- glucosamine, N-acetyl-D-glucosamine, mannosamine, N-acetyl mannosamine, galactosamine, N-acétyl galactosamine, vitamine B3 et ses dérivés, le niacinamide, déhydro-acétate de sodium, l'acide déhydroacétique et ses sels, phytosterols, composés d'acide salicylique, hexamidines, composés dihydroxyproline de dialkanoyl, extraits et dérivés de soja, equol, isoflavones, flavonoïdes, phytantriol, farnesol, géraniol, bisabolol, peptides et leurs dérivés, di -, tri -, tétra -, penta -, et hexapeptides et leurs dérivés, lys-thr-thr-lys-ser, palmitoyl-lys-thr-thr-lys-ser, carnosine, composés d'acide aminé N-acyl, rétinoïdes, propionate de rétinyl, rétinol, palmitate de rétinyl, acétate de rétinyl, rétinal, acide rétinoïque, vitamines hydrosolubles, ascorbates, vitamine C, glucoside ascorbyl, palmitate ascorbyl, magnésium ascorbyl phosphate, sodium ascorbyl phosphate, vitamines et leurs sels et dérivés, provitamines et leurs sels et dérivés, ethyl panthenol, vitamine B et ses dérivés, vitamine B1, vitamine B2, vitamine B6, vitamine B12, vitamine K et ses dérivés, acide pantothénique et ses dérivés, éther éthylique de pantothenyl, panthenol et ses dérivés, panthenol ethylique, dexpanthenol, biotine, acides aminés et leurs sels et les dérivés, acides aminés hydrosolubles, asparagine, alanine, indol, acide glutamique, vitamines insolubles dans l'eau, vitamine A, vitamine E, vitamine F , vitamine D et ses composés, mono-, di -, et triterpénoïdes, bêta-ionol, cedrol, et leurs dérivés, acides aminés insolubles dans l'eau, tyrosine, tryptamine, matériaux particulaires, hydroxytoluène butylé, hydroxyanisole butylé, allantoine, nicotinate de tocophérol, tocophérol, esters de tocophérol, palmitoyl-gly-his-lys, phytostérol, hydroxy acides, acide glycolique, acide lactique, acide lactobionique, kétoacides, acide pyruvique, acide phytique, acide lysophosphatidique, stilbenes, cinnamates, resveratrol, kinétine, zeatin, dimethylaminoethanol, peptides naturels, les peptides de soja, sels de sucres acides, gluconate de manganèse, gluconate de zinc, olamine de piroctone, 3,4,4'- trichlorocarbanilide, triclocarban, pyrithione de zinc, hydroquinone, acide kojique, acide ascorbique, magnésium ascorbyl phosphate, ascorbyl glucoside, pyridoxine, l'aloe vera, les alcools de terpène, allantoine, bisabolol, glycyrrhizinate dipotassique, acide de glycérol, de sorbitol, de pentaerythritol, de pyrrolidone et ses sels, dihydroxyacetone, erythrulose, glycéraldéhyde, tartaraldehyde, l'essence de clou de girofle, le menthol, le camphre, l'essence d'eucalyptus, eugénol, le lactate de menthyle, le distillat d'hamamélis, copolymère d'eicosene et vinyl pyrrolidone, iodopropyl butylcarbamate, un polysaccharide, un acide gras essentiel, un salicylate, un acide glycyrrhétinique, des caroténoïdes, des céramides et des pseudo-céramides, un lipide complexe, les huiles en générale d'origine naturelle telles le beurre de karité, l'huile d'abricot, l'huile d'onagre, l'huile de pruneau, l'huile de palme, l'huile de monoï, l'huile de kahai, hydroquinone, 1'HEPES, la procystéine, l'O-octanoyl-6-D-maltose, le sel disodique d'acide méthyl glycine diacétique, les stéroïdes tels que la diosgénine et les dérivés de la DHEA, la DHEA déhydroépiandrostérone et/ou un précurseur ou dérivé chimique ou biologique, le N-éthylcarbonyl-4-para-aminophénol, les extraits de myrtille, les phytohormones, les extraits de levure *Saccharomyces cerevisiae,* les extraits d'algues, les extraits de soja, de lupin, de maïs et/ou de pois, l'alvérine et ses sels, en particulier le citrate d'alvérine, les extraits de petits houx et de marron d'inde et leurs combinaisons, un inhibiteur de métalloprotéinases, les extraits de *Schinus molle.*

Dans tous les cas, l'homme de métier veillera à ce que ces adjuvants ainsi que leurs proportions soient choisis de telle manière à ne pas nuire aux propriétés avantageuses recherchées de la composition selon l'invention.

Dans un mode de réalisation préférentiel selon l'invention, la composition cosmétique comprend entre 10⁻⁶ et 50% en poids par rapport au poids total de la composition de principe actif.

La quantité efficace de principe actif ou d'extrait végétal correspond à la quantité nécessaire pour obtenir le résultat recherché, à savoir, améliorer l'hydratation et la fonction barrière de l'épiderme, prévenir et/ou lutter contre la sécheresse de la peau et des muqueuses et contre les manifestations du vieillissement cutané, aider au processus de cicatrisation et dépigmenter la peau. L'activité anti-inflammatoire est exclue du champ d'activité dermatologique revendiqué.

Selon un mode de réalisation avantageux de l'invention, la quantité efficace ou la teneur en principe actif dans la composition selon l'invention est de 10⁻⁶ à 25 % en poids par rapport au poids total de la composition, de préférence encore est de 10⁻⁶ à 10 % en poids par rapport au poids total de la composition.

Les quantités efficaces indiquées sont basées sur les résultats donnés précisément dans les exemples 21 à 23. Ainsi, en fonction de la concentration en sucrose-esters de l'extrait, de la dilution effectuée si nécessaire, la quantité efficace varie dans les proportions indiquées ci-avant.

Dans un mode de réalisation plus avantageux encore selon l'invention, la composition cosmétique est adaptée à une administration topique et se présente notamment sous forme d'une solution aqueuse, hydro-alcoolique ou huileuse ou sous la forme d'une émulsion huile-dans-eau, eau-dans-huile ou émulsions multiples ou sous forme de crèmes, de suspensions, ou encore de poudres ; ladite composition pouvant être plus ou moins fluide ou solide et avoir l'aspect d'une crème, d'une lotion, d'un lait, d'un shampooing, d'un sérum, d'une pommade, d'un gel, d'une pâte, d'une mousse ou d'un stick.

Un troisième objet selon l'invention consiste en un procédé de traitement non-thérapeutique pour améliorer l'aspect de la peau, des muqueuses ou des phanères, pour prévenir et/ou lutter contre la sécheresse de la peau et des muqueuses, pour prévenir et/ou à lutter contre les signes cutanés du vieillissement et/ou du photo-vieillissement, pour lutter contre la perte d'élasticité et de fermeté de la peau et pour dépigmenter la peau, procédé consistant à appliquer sur la surface de la peau et/ou des cheveux, une quantité efficace d'une composition cosmétique selon l'invention telle que définie ci-avant.

Il est à noter que l'activité dépigmentante de la composition cosmétique selon l'invention présente une activité inhibitrice sur la production de la mélanine.

Une quantité efficace de sucrose-esters selon l'invention, seuls ou en association avec d'autres principes actifs, peut être utilisée dans le traitement dermatologique des pathologies impliquant une sécheresse pathologique de la peau, des muqueuses ou des phanères et pour favoriser la cicatrisation.

La quantité efficace est également basée sur les résultats donnés précisément dans les exemples 21 à 23. Les tests notamment sur le collagène III, le collagène IV et la fibrilline-1 permettent d'identifier une quantité efficace d'au moins 10⁻⁶ % en poids par rapport au poids total de la composition.

Un quatrième objet selon l'invention consiste en une composition nutracosmétique à ingérer comprenant une quantité efficace comprise entre 10⁻⁶ et 50% en poids par rapport au poids total de la composition de sucrose-esters selon l'invention, seuls ou en association avec d'autres principes actifs, destinée à améliorer l'aspect de la peau, des muqueuses ou des phanères, à prévenir et/ou lutter contre la sécheresse de la peau et des muqueuses, à prévenir et/ou à lutter contre les signes cutanés du vieillissement et/ou du photo-vieillissement.

Enfin, un cinquième objet selon l'invention consiste en un procédé de préparation d'un mélange de sucrose-esters provenant d'un extrait végétal.

Plus particulièrement selon l'invention on utilise le calice de *Physalis peruviana*.

Toute méthode d'extraction et de purification connue de l'homme du métier peut être utilisée afin de préparer l'extrait végétal selon l'invention comprenant des sucrose-esters tel que décrits précédemment et possédant une activité biologique sur la peau, les muqueuses et les phanères.

Le procédé de préparation d'un extrait végétal selon l'invention est décrit plus précisément ci-dessous.

La plante et parties de plante peuvent être récoltées par cueillette dite sauvage, par mise en culture conventionnelle, par mise en culture hors sol ou encore en culture hydroponique.

De façon non exhaustive, le procédé de préparation de l'extrait brut de l'extrait végétal peut être effectué par des procédés d'extraction végétale solide/liquide plus ou moins conventionnels, utilisant principalement des solvants organiques, bien connus de l'homme de l'art. Le procédé d'extraction peut être à simple contact en discontinu, en semi-continu ou en continu. Il peut être aussi à étages multiples, à co-courant ou à contre-courant. Il peut être assisté par ultrasons, par micro-ondes, par induction thermomagnétique, par champs électriques pulsés ou encore sous pression.

La matrice végétale peut-être préparée au préalable par séchage puis par broyage ou cryobroyage, par flash détente ou encore par détente instantanée contrôlée. Aussi, la matrice végétale peut aussi être digérée par des enzymes afin de libérer le maximum de molécules d'intérêt.

Les solvants pouvant être utilisés pour extraire les sucrose-esters sont :
- des hydrocarbures, aliphatiques linéaires ou ramifiés, aromatiques ou cycliques tels que l'hexane, le cyclohexane, ...
- des hydrocarbures halogénés, tels que le dichloroéthylène, le chloroforme, ... ;
- des alcools tels que le méthanol, l'éthanol, le propanol, l'isopropanol ou le butanol, ...
- des glycols tels que l'éthylène glycol, le propylène glycol, les 1,2 et 1,3 propanediols, le butylène glycol, le glycérol, ...
- des cétones tels que l'acétone, le méthyl isobutyl cétone, la méthyl éthyl cétone, ...
- des éthers aliphatiques ou cycliques tels que l'éther diéthylique, le méthyl tertio butyl éther, l'éthyl tertio butyl éther, le TétraHydroFurane (THF), le méthyl THF, ......
- des éthers de glycol tels que le 2-méthoxy-1-méthyléthyl acétate, le 2-(2-butoxyéthoxy) éthanol et son acétate, ...
- des esters, d'acides à chaîne plus ou moins longue et d'alcools à chaîne plus ou moins longue tels que l'acétate d'éthyle, le lactate d'éthyle, le propionate d'éthyle, l'oléate d'éthyle, le stéarate de méthyle, l'oléate d'oleyle, ...
- des liquides ioniques tels que le 1-(4-sulfobutyl)-3-methylimidazolium hydrogène sulfate, le 1-Ethyl-3-methylimidazolium bis (trifluoromethanesulfonyl) imide, ...
- des fluides supercritiques tels que le dioxyde de carbone avec ou sans co-solvants ;
- des agro-solvants tels que des huiles végétales, des terpènes comme le limonène ou le pinène,
- une combinaison de ces solvants.

L'extrait ainsi obtenu peut être purifié par différentes méthodes, notamment par extraction liquide - liquide, par centrifugation, par adsorption (par exemple par décoloration au charbon actif ou à la terre décolorante), par sublimation, par cristallisation , par chromatographie préparative, par distillation notamment par distillation moléculaire de type centrifuge ou à film raclée ou encore par filtration membranaire.

Selon la technique de purification utilisée, on effectuera éventuellement une étape ultérieure de filtration et de désolvantation.

Dans un mode de réalisation préférentiel, l'extrait végétal brut est obtenu avec l'un des solvants suivants : le cyclohexane, l'acétate d'éthyle et le dioxyde de carbone supercritique avec une addition d'un co-solvant tel qu'un alcool, par exemple un alcool aliphatique comme l'éthanol, ou une huile végétale, de préférence raffinée ou encore un ester comme le caprylate/caprate de glycérol.

Ainsi, le procédé de préparation d'un mélange de sucrose-esters provenant_d'un extrait végétal selon l'invention comprend les étapes suivantes :
- séchage de la partie de plante,
- broyage,
- extraction avec le dioxyde de carbone supercritique sans ou avec co-solvant tels qu'un alcool ou une huile végétale ou encore un ester, puis
- éventuellement une purification avec une ou plusieurs méthodes appropriées.

D'autres avantages et caractéristiques de l'invention apparaîtront mieux à la lecture des exemples donnés à titre illustratif et non limitatif.

### Exemple 1 : Préparation d'un extrait végétal à partir du calice de Physalis peruviana

Les calices de *Physalis peruviana,* séparés préalablement des fruits et séchés, sont broyés dans un broyeur à couteaux comportant une grille de 1 mm, ce qui permet d'obtenir une poudre dont la taille des particules se situe majoritairement vers 500 µm.

La poudre (50 g) ainsi obtenue est placée dans une cartouche en cellulose, cette cartouche est placée dans un extracteur type Soxhlet.

Le solvant utilisé pour extraire est l'éthanol 96% (50 ml), avec une vingtaine de cycles d'extraction.

L'extrait en milieu solvant est évaporé au rotavapor afin d'éliminer le solvant.

Il est ainsi obtenu 11,0 g d'extrait de calices de *Physalis peruviana* contenant 57% de sucrose-esters.

### Exemple 2 : Préparation d'un extrait végétal à partir du calice de Physalis peruviana

Les calices de *Physalis peruviana,* séparés préalablement des fruits et séchés, sont broyés dans un broyeur à couteaux comportant une grille de 1 mm, ce qui permet d'obtenir une poudre dont la taille des particules se situe majoritairement vers 500 µm.

La poudre ainsi obtenue (50 g) est placée dans une cartouche en cellulose, cette cartouche est placée dans un extracteur type Soxhlet.

Le solvant utilisé pour extraire est l'acétate d'éthyle, avec une vingtaine de cycles d'extraction. L'extrait ainsi obtenu en milieu solvant peut-être purifié par une extraction liquide - liquide sur colonne avec de l'eau, à une température comprise entre 20°C et 65°C, à contre-courant afin d'éliminer des molécules hydrosolubles. La phase organique comprenant l'acétate d'éthyle et l'extrait est ensuite évaporé au rotavapor afin d'éliminer le solvant.

L'extrait ainsi lavé est exsangue de polyphénols, de whitanolides et de sucres libres.

Il est ainsi obtenu 6,2 g d'extrait de calices de *Physalis peruviana* contenant 89,0% de sucrose-esters.

### Exemple 3 : Préparation d'un extrait végétal à partir du calice de Physalis peruviana

Les calices de *Physalis peruviana,* séparés préalablement des fruits et séchés, sont broyés dans un broyeur à couteaux comportant une grille de 1 mm, ce qui permet d'obtenir une poudre dont la taille des particules se situe majoritairement vers 500 µm. La poudre ainsi obtenue (500 g) est placée dans un extracteur à fluides supercritiques.

Le solvant utilisé pour extraire est le dioxyde de carbone avec une addition d'un co-solvant tel qu'un alcool aliphatique comme l'éthanol.

Les paramètres d'extraction sont pour une pression comprise entre 75 et 200 bars, une température comprise entre 35 et 50°C, un débit de CO₂ compris entre 2 et 20 kg/h, ainsi que 1 à 5% d'éthanol par rapport au CO₂.

L'extrait ainsi obtenu en milieu solvant est décoloré au charbon actif ou à la terre décolorante, cristallisé à froid puis filtré et évaporé.

Il est ainsi obtenu 85,5 g d'extrait de calices de *Physalis peruviana* contenant 87,6% de sucrose-esters.

Si nécessaire, l'extrait ainsi obtenu pourra être purifié par distillation moléculaire de type centrifuge ou à film raclé, afin d'obtenir un extrait de calices de *Physalis peruviana* à 99,9% de pureté chromatographique.

### Exemple 4 : Purification des sucrose-esters à partir d'un extrait végétal obtenu à partir du calice de Physalis peruviana

Un extrait de calice de physalis (1 g) obtenu selon l'exemple 1 est solubilisé dans 6 ml d'un mélange de solvant CH₃CN/H₂O 1:1. La solution est filtrée sur une cartouche par Extraction sur Phase Solide (SPE) (C18, 10 g) qui est rincée avec 10 mL d'un même mélange CH₃CN/H₂O 1:1.

L'éluat obtenu est associé au solvant de charge pour obtenir la fraction 1 (300 mg).

La cartouche chargée est alors successivement éluée selon le gradient de solvant CH₃CN/H₂O 7:3 (20 mL) et 1:0 (20 mL) pour obtenir les fractions 2 (500 mg) et 3 (100 mg).

Les fractions 1, 2 et 3 sont analysées par HPLC-DAD-DEDL et sont comparées au chromatogramme de l'extrait initial.

La fraction 1 est constituée des composés polyphénoliques, des withanolides et physalines et de composés polaires tels que des sucres.

La fraction 2 est composée des sucrose-esters.

La fraction 3 est une fraction lipidique.

La fraction 2 (500 mg) est soumise à un fractionnement par HPLC préparative (C18, 50x150 mm, 5µm) selon un gradient de solvant H₂O/CH₃CN (de 3:7 à 0:1 en 35 min.) pour obtenir les fractions :
- Fraction IIIb (élution entre 4 et 14 min.),
- Fraction I (élution entre 14 et 18 min. ; 230 mg),
- Fraction II (elution entre 18 et 25 min. ; 200 mg) et
- Fraction IIIa (entre 25 et 35 min.).

Les fractions Fraction IIIa et IIIb sont rassemblées pour obtenir la fraction Fraction III (50 mg).

Composition des fractions obtenues :
Fraction I : Composé 1, 3-*O*-(2-methyl-1-oxopropyl)-*β-D*-fructofuranosyle (1→2)-3,4-bis(2-methylpropanoate) -2-decanoate-*α-D*-glucopyranoside.
Fraction II : Composé 2, 3-*O*-(3-methyl-1-oxobutyl)-*β-D*-fructofuranosyle (1→2)-3,4-bis(2-methylpropanoate)-2-decanoate-*α-D*-glucopyranoside.
Fraction III : sucrose-esters autres que précédemment et comprenant notamment
   - le 3-O-(3-methyl-1-oxobutyl)-β-D-fructofuranosyle(1→2)-3-(2-methylpropanoate)-2-decanoate-α-D-glucopyranoside,
   - le 3-O-(2-methyl-1-oxopropyl)-β-D-fructofuranosyle(1→2)-3,6-bis(2-methylpropanoate)-2-decanoate-α-D-glucopyranoside.
   - le 3-O-(2-methyl-1-oxopropyl)-β-D-fructofuranosyle(1→2)-3-(3-methylbutanoate)-2-decanoate-α-D-glucopyranoside
   - le 3-O-(2-methyl-1-oxopropyl)-β-D-fructofuranosyle(1→2)-3,4-bis(2-methylpropanoate)-2-nonanoate-α-D-glucopyranoside,
   - le 3-O-(2-methyl-1-oxopropyl)-β-D-fructofuranosyle(1→2)-3,4-bis(2-methylpropanoate)-2-octanoate-α-D-glucopyranoside,

### Exemple 5 : Effets des fractions I, II et III sur le profil d'expression de cellules cutanées

Les effets des fractions I, II et II ont été recherchés par analyse transcriptomique sur les modèles de kératinocytes épidermiques humains normaux et de fibroblastes dermiques humains. Cette analyse globale d'expression de transcrits a été réalisée après 4 à 24 heures d'incubation en utilisant la plateforme Affymetrix GeneAtlas^{™} et la puce « full transcriptome humain » U219 contenant 36000 transcrits et variants.

### 1.Modèles biologiques

### Kératinocytes épidermiques humains normaux (NHEK)

| | |
|---|---|
| - Type cellulaire : | Kératinocytes épidermiques humains normaux (NHEK) |
| - Conditions de culture : | 37°C, 5% CO₂ |
| - Milieu de culture : | Keratinocyte-SFM complémenté avec |
| | Epidermal Growth Factor (EGF) 0.25 ng/ml |
| | Extrait Pituitaire (EP) 25 µg/ml |
| | Gentamycine 25 µg/ml |
| - Milieu d'essai : | Keratinocyte-SFM complémenté avec |
| | Gentamycine 25 µg/ml |

### Fibroblastes dermiques humains normaux (NHDF)

| | |
|---|---|
| - Type cellulaire : | Fibroblastes dermiques humains normaux (NHDF), |
| - Conditions de culture : | 37°C, 5% CO₂ |
| - Milieu de culture : | DMEM complémenté avec |
| | L-glutamine 2 mM |
| | Pénicilline 50 U/ml - Streptomycine 50 µg/ml |
| | Sérum de veau foetal (SVF) 10% |
| - Milieu d'essai : | DMEM complémenté avec |
| | L-glutamine 2 mM |
| | Pénicilline 50 U/ml - Streptomycine 50 µg/ml |
| | SVF 1 % |

### 2. Composés testés

| **Composé testé** | **Aspect/Stockage** | **Solution stock** | **Concentration testée** |
|---|---|---|---|
| Fraction I | ▪ Pâte | 50 mg/ml en DMSO | 0.8 µg/ml |
| | ▪ Stockage à +4°C | | |
| Fraction II | ▪ Pâte | 50 mg/ml en DMSO | 0.8 µg/ml |
| | ▪ Stockage à +4°C | | |
| Fraction III | ▪ Pâte | 50 mg/ml en DMSO | 0.8 µg/ml |
| | ▪ Stockage à +4°C | | |

### 3 Culture et traitement

Les cellules ont été ensemencées et cultivées en milieu de culture pendant 24 heures puis incubées dans leur milieu d'essai pendant 24 heures supplémentaires. Le milieu a ensuite été remplacé par le milieu d'essai contenant ou non (témoin) les composés à l'essai et les cellules ont été incubées pendant 4 heures ou 24 heures. Toutes les conditions ont été réalisées en n=3. A la fin de l'incubation, les surnageants de culture ont été éliminés et les tapis cellulaires ont été rincés avec une solution de PBS. Les plaques ont été immédiatement congelées à sec à -80°C.

### 4. Principe d'utilisation de la puce Affymetrix^{®} U219 - Analyse de l'expression différentielle

### - Préparation des cibles

Les ARN totaux de chaque échantillon ont été extraits à l'aide de *TriPure Isolation Reagent*^{®} selon le protocole préconisé par le fournisseur. La quantité et la qualité des ARN ont été évaluées par électrophorèse capillaire (Bioanalyzer 2100, Agilent).

L'amplification des ARN et la synthèse des analogues d'ARN biotinylés (ARNa) ont été réalisées à l'aide du kit « GeneChip 3'IVT Express » (Affymetrix^{®}). Les ADN complémentaires (ADNc) simple brin ont été synthétisés par transcription inverse des ARN totaux en présence d'oligo(dT). Par action d'une ADN polymérase, un ADNc double brin (ADNdb) a ensuite été synthétisé. Des ARN biotinylés ont ensuite été synthétisés à partir des ADNc et en présence d'analogues de ribonucléotides biotinylés. Après une étape de purification sur billes magnétiques, permettant d'éliminer les sels, enzymes et nucléotides libres, les ARN biotinylés ont été hydrolysés en fragments de 35 à 200 nucléotides avec un pic à 100-120 nucléotides (cibles fragmentées).

Des contrôles qualité des ARN biotinylés synthétisés ont été réalisés par électrophorèse capillaire (Bioanalyzer 2100, Agilent), avant et après fragmentation.

### - Protocole d'hybridation et de marquage

Cette étape a été réalisée à l'aide du kit « GeneAtlas^{™} hybridization, wash and stain kit for 3'IVT arrays » (Affymetrix^{®}).

L'hybridation des ARNa fragmentés sur la puce Affymetrix^{®} U219 (36 000 transcrits et variants) a été réalisée sur la station d'hybridation « GeneAtlas^{™} fluidics station » (Affymetrix^{®}) pendant 20 heures à 45°C. Des sondes de contrôle de l'hybridation (BioB, BioC et BioD) et des ARN polyA contrôles (Lys, Phe et Dap) ont été ajoutées au cours de l'hybridation. Les sondes de contrôle de l'hybridation, directement couplées à la phycoérythrine, permettent de s'assurer de l'efficacité de l'hybridation indépendamment de l'étape de marquage. Les ARN poly-A contrôles sont quant à eux utilisés pour valider la qualité du marquage.

Le marquage a ensuite été effectué en 3 étapes (+ étapes de lavage intermédiaires) :
- Fixation du complexe streptavidine - phycoérythrine sur les ARNa hybridés sur la puce,
- Reconnaissance de ce complexe par un anticorps anti-streptavidine couplé à la biotine,
- Ajout de la streptavidine couplée à la phycoérythrine permettant une amplification du signal.

### 5 Traitement des données

Les données brutes ont été transférées et traitées sous le logiciel Microsoft Excel.

Les comparaisons intergroupes ont été réalisées à l'aide du test T de Student bilatéral non apparié. Les analyses statistiques peuvent être interprétées si n≥5 ; cependant pour n<5, les données calculées ne sont fournies qu'à titre indicatif.

### Formules utilisées :

| | |
|---|---|
| Erreur standard de la moyenne : | esm = écart-type (Sd)/√n |
| | L'erreur standard de la moyenne (esm) représente l'écart de la moyenne de l'échantillon par rapport à la moyenne de la vraie population. L'esm est calculé en divisant le Sd par la racine carrée de la taille de l'échantillon. |
| Pourcentage de viabilité : | viabilité (%) = (DO _{composé} / DO _{témoin}) × 100 |

### 6 Résultats

Une analyse des « fold change » dans le logiciel Excel a été effectuée puis une analyse fonctionnelle a été réalisée à l'aide du logiciel IPA (Interactive Pathway Analysis) d'Ingenuity. Cette analyse permet de regrouper les gènes significativement modulés dans des fonctions (processus) biologiques

### Analyse des fibroblastes (NHDF)

L'analyse des profils transcriptomiques des fibroblastes traités par les fractions (I, II et III) a montré une modulation d'expression de gènes, surtout avec la fraction II.

Au niveau de la fraction II, la modulation de l'expression du gène codant pour la fibrilline 1 (FBN1) est très intéressante. En effet, l'expression de ce gène est augmentée de façon significative aux temps 4 heures (X 3.27) et 24 heures (X 8.24).

La fibrilline 1 (constituant majeur de la matrice extracellulaire) est sécrétée par les fibroblastes et constitue un composant important des microfibrilles. Elle est impliquée notamment dans l'assemblage des fibres d'élastine, et joue un rôle important dans l'élasticité de la peau.

### Analyse des kératinocytes (NHEK)

L'analyse des profils transcriptomiques des kératinocytes traités par les 3 fractions (I, II et III) a montré une modulation d'expression de gènes, surtout avec la fraction III.

Les traitements des kératinocytes par les 3 fractions (I, II et III) ont induit des inhibitions d'expression de gènes codant pour des protéines de différenciation du kératinocyte : KRT1, IVL, DSG1, DSC1, SPRR2A, SPRR2E, SPRR1A, SPRR2D, CALML5, SBSN et KRTDAP.

Cet effet permet de dire que les fractions I, II et III ont un effet anti différenciant sur les kératinocytes.

Dans les exemples qui suivent, l'ingrédient actif selon l'invention peut être présent dans les différentes compositions en une quantité efficace comprise entre 10⁻⁶ à 3% en masse de la composition.

### Exemple 6 : Compositions pour administration par voie orale

L'extrait de calice de *Physalis peruviana,* contenant les sucrose-esters, est intégré à des compositions orales, dans des compositions permettant l'administration de 50 mg à 200 mg d'extrait par jour.
1) Composition anti-âge sous forme de capsules molles
- Extrait de calice de *Physalis peruviana* selon exemple

| | |
|---|---|
| 1 ou 2 ou 3 | 30 mg |
| - Huile d'Argan | 60 mg |
| - Huile de germes de blé riche en insaponifiable | 300 mg |
| - Vitamine du groupe B (B1, B2, B3, B5, B6, B9, B12) | QSP 100% des AJR |
| - Tocotriènols | QSP 50% des AJR |
| - Vitamine E | |
| - Cire d'abeilles | |
| - Lécithine de Soja | |
| - Gélatine alimentaire | |
| - Glycérine | QSP 1 capsule molle |

Cette composition est administrée de 4 à 6 capsules par jour.
2) Composition fermeté de la peau sous forme de comprimés
- Extrait de calice de *Physalis peruviana* selon exemple

| | |
|---|---|
| 1 ou 2 ou 3 | 25 mg |
| - Extraits de céréales (blé, sarrasin, riz, quinoa) riches en acides aminés soufrés | 200 mg |
| - Zinc sous forme de chélate | QSP 100% des AJR |
| - Vitamine C | QSP 50% des AJR |
| - Glycosaminoglycanes issus de cartilages de poissons | 200 mg |
| - Glucidex IT 19 (agent de compression) | QSP 1 comprimé de 800 mg |

Cette composition est administrée de 5 à 8 comprimés par jour.
3) Exemple en barre céréalière goût chocolat
- Extrait de calice de *Physalis peruviana* selon exemple

| | |
|---|---|
| 1 ou 2 ou 3 | 200 mg |
| - Lycopène | 6 mg |
| - Astaxanthine | 4 mg |
| - Fucoxanthine | 4 mg |
| - Lutéine sous forme micro-encapsulée | 4 mg |
| - Tocotriènols micro-encapsulés | QSP 100% AJR en Vitamine E |
| - chocolat noir, oligo-fructose, sucre, sirop de fructose, cacao maigre en poudre, céréales croustillantes, lait écrémé en poudre, amandes, glycérol, sorbitol, huiles végétales, sirop de glucose, arôme, lait concentré sucré, lécitine de soja, mono et diglycérides d'acides gras, sirop caramélisé, maltodextrine, sel, sorbate de potassium, alpha tocophérol | QSP une barre de 50 g |

Cette composition est administrée une fois par jour.
4) Exemple en boisson lactée goût vanille
- Extrait de calice de *Physalis peruviana* selon exemple

| | |
|---|---|
| 1 ou 2 ou 3 | 200 mg |
| - Extrait de thé vert riche en polyphénols | 100 mg |
| - Vitamine du groupe B (B1, B2, B3, B5, B6, B9, B12) | QSP 100% des AJR |
| - Zinc, magnésium, sélénium | QSP 100% des AJR |
| Lait écrémé en poudre, arôme, fructose, blanc d'oeuf, grains de vanille épuisée, sucre, caramel, béta carotène, gomme xanthane, asparthame, acésulfame de potassium, lécithine de soja, maltodextrine. | QSP un sachet de 30 g |
| Cette composition est administrée une fois par jour. | |

### Exemple 7 : Composition cosmétique - Crème de jour pour le visage

| **Noms INCI** | **% en masse** |
|---|---|
| **Phase A** | |
| Eau | Qsp 100 |
| Carbomère | 0,25 |

| **Phase B** | |
|---|---|
| Butylène glycol | 2,00 |
| Phenoxyethanol | qs |

| **Phase C** | |
|---|---|
| Steareth-2 | 0,40 |
| Steareth-10 | 1,20 |
| Cetearyl alcohol & Dicetyl phosphate & Ceteth 10 phosphate | 4,00 |
| Cetearyl Alcohol | 1,00 |
| Azone | 2,50 |
| Cyclohexasiloxane & Cyclopentasiloxane | 2,00 |
| Ethylhexyl succinate | 7,00 |

| **Phase D** | |
|---|---|
| Sorbate de potassium | 0,10 |

| **Phase E** | |
|---|---|
| Eau | 3,00 |
| Hydroxyde de sodium | 0,40 |

| **Phase F** | |
|---|---|
| Ingrédient actif selon l'invention (selon exemple 1 ou 2 ou 3) | 2,00 |

Mode opératoire:
Peser la phase A et laisser gonfler sans agitation pendant 30 mn. Mettre la phase A à chauffer à 75 °C au bain-marie. Peser et mélanger la phase B. Peser la phase C et mettre à chauffer à 75°C au bain-marie. Rajouter la phase B dans la phase A. Bien mélanger. Sous agitation verser la phase C dans la phase A+B. Bien homogénéiser. Rajouter la phase D, extemporanément. Rajouter la phase E, bien homogénéiser. Rajouter la phase F, bien homogénéiser.

### Exemple 8 : Composition cosmétique - Forme gel pour le visage

| **Noms INCI** | **% en masse** |
|---|---|
| **Phase A** | |
| Eau | Qsp 100 |
| Cetyl hydroxyethylcellulose | 0,30 |

| **Phase B** | |
|---|---|
| Carbomère | 0,40 |
| Eau | 20,00 |

| **Phase C** | |
|---|---|
| Glycérine | 3,00 |
| Ethyl & Methyl & Propyl parabens | 0,30 |

| **Phase D** | |
|---|---|
| Huile minérale | 4,00 |
| Polysorbate 20 | 1,00 |
| C12-15 Alkyl Benzoate | 2,00 |
| C10-30 Alkyl Acrylate cross polymer | 0,30 |

| **Phase E** | |
|---|---|
| Sorbate de potassium | 0,10 |

| **Phase F** | |
|---|---|
| Eau | 5,00 |
| Hydroxyde de sodium | 0,50 |

| **Phase G** | |
|---|---|
| Ingrédient actif selon l'invention (selon exemple 1 ou 2 ou 3) | 1,00 |

Mode opératoire :
Disperser la Phase A sous agitation. Saupoudrer l'Ultrez 10 dans l'eau, laisser gonfler 30 minutes. Chauffer la Phase C jusqu'à dissolution complète. Mélanger la Phase A avec la Phase B. Rajouter C dans la Phase (B+A). Ajouter la Phase D, sous agitation, dans la Phase (A+B+C). Rajouter la Phase E. Neutraliser avec la Phase F. Ajouter la Phase G et mélanger.

### Exemple 9 : Composition cosmétique - Forme sérum

| **Noms INCI** | **% en masse** |
|---|---|
| **Phase A** | |
| Eau | Qsp 100 |
| Carbomère | 0,25 |

| **Phase B** | |
|---|---|
| Butylène Glycol | 3,00 |
| Phenoxyethanol | 0,20 |

| **Phase C** | |
|---|---|
| Polysorbate 20 | 0,50 |
| Cetearyl Ethylhexanoate | 2,00 |
| PPG-3 Benzyl Ether Myristate | 0,50 |
| Acrylates/C 10-30 Alkyl polymer | 0,20 |
| Acrylates cross | |
| Cyclopentasiloxane | 1,00 |

| **Phase D** | |
|---|---|
| Sorbate de potassium | 0,10 |

| **Phase E** | |
|---|---|
| Hydroxyde de sodium | 0,45 |
| Eau | 4,00 |

| **Phase F** | |
|---|---|
| Ingrédient actif selon l'invention (exemple 1 ou 2 ou 3) | 1,00 |

| **Phase G** | |
|---|---|
| Fragrance | 0,10 |

Mode opératoire :
Phase A : Saupoudrer le carbomer dans l'eau, laisser gonfler 15 minutes. Mélanger la Phase B. Verser la Phase B dans la Phase A, homogénéiser. Puis Peser la Phase C, mélanger et rajouter dans la Phase A+B, sous agitation. Laisser gonfler 1 heure. Extemporanément rajouter la Phase D dans la phase précédente sous agitation. Neutraliser avec la Phase E. Mettre sous agitation.
Ensuite rajouter la Phase F. Laisser mélanger au moins 1 heure sous agitation puis rajouter la Phase G. Bien mélanger.

### Exemple 10 : Crème de nuit pour visage

| **Noms INCI** | **% en masse** |
|---|---|
| **Phase A** | |
| Eau | Qsp 100 |
| Carbomère | 0,40 |

| **Phase B** | |
|---|---|
| Glycérine | 3,00 |
| Ethyl & Methyl & Propyl parabens | 0,30 |

| **Phase C** | |
|---|---|
| Cetearyl Alcohol & polysorbate 20 | 1,0 |
| Cetearyl Alcohol | 1,00 |
| PPG-3 Benzyl Ether Myristate | 1,0 |
| Dimethicone | 2,50 |
| Isotridecyl Isononanoate | 5,00 |

| **Phase D** | |
|---|---|
| Sorbate de potassium | 0,10 |

| **Phase E** | |
|---|---|
| Hydroxyde de sodium | 0,40 |
| Eau | 4,00 |

| **Phase F** | |
|---|---|
| Ingrédient actif selon l'invention (selon exemple 1 ou 2 ou 3) | 1,00 |

| **Phase G** | |
|---|---|
| Fragrance | 0,10 |
| | |

Mode opératoire : Peser Phase A et laisser gonfler pendant 30 minutes. Chauffer Phase A dans un bain d'eau à 75°C ; Chauffer Phase B jusqu'à solubilisation complète. Ajouter Phase B à Phase A. Chauffer Phase C dans un bain d'eau à 75°C. Ajouter la Phase C à la Phase A+B sous agitation. Ajouter Phase D et bien homogénéiser. Neutraliser avec Phase E à 55°C. Ajouter Phase F puis Phase G et bien homogénéiser.

### Exemple 11 : Crème pour le corps.

| **Noms INCI** | **% en masse** |
|---|---|
| **Phase A** | |
| Eau | Qsp 100 |
| Carbomère | 0,4 |

| **Phase B** | |
|---|---|
| Glycérine | 3,00 |
| Ethyl & Methyl & Propyl parabens | 0,30 |

| **Phase C** | |
|---|---|
| Sorbitan stéarate | 2,00 |
| Mineral Oil | 4,00 |
| PPG-3 Benzyl Ether Adipate | 1,00 |
| Glyceryl stéarate & PEG 100 stéarate | 3,00 |

| **Phase D** | |
|---|---|
| Sorbate de potassium | 0,10 |

| **Phase E** | |
|---|---|
| Hydroxyde de sodium | 0,40 |
| Eau | 4,00 |

| **Phase F** | |
|---|---|
| Ingrédient actif selon l'invention (selon exemple 1 ou 2 ou 3) | 2,00 |

| **Phase G** | |
|---|---|
| Fragrance | 0,10 |

Mode opératoire : Peser Phase A et laisser gonfler pendant 30 minutes. Chauffer Phase A dans un bain d'eau à 75°C. Chauffer Phase B jusqu'à solubilisation complète. Ajouter Phase B à Phase A. Chauffer Phase C dans un bain d'eau à 75°C. Ajouter Phase C à Phase A+B sous agitation. Ajouter Phase D et bien homogénéiser. Neutraliser avec Phase E à 55°C. Ajouter Phase F puis Phase G et bien homogénéiser.

### Exemple 12: Lotion

| **Noms INCI** | **% en masse** |
|---|---|
| **Phase A** | |
| Eau | Qsp 100 |

| **Phase B** | |
|---|---|
| Butylène Glycol | 5,00 |
| Phenoxyethanol | 0,20 |

| **Phase C** | |
|---|---|
| Polysorbate 20 | 2,00 |
| PPG-3 Benzyl Ether Myristate | 0,10 |

| **Phase D** | |
|---|---|
| Sorbate de potassium | 0,10 |

| **Phase E** | |
|---|---|
| Ingrédient actif selon l'invention (selon exemple 1 ou 2 ou 3) | 1,00 |

| **Phase F** | |
|---|---|
| Fragrance | 0,10 |

Mode opératoire : Peser Phase A. Peser Phase B et mélanger. Ajouter Phase B à Phase A sous agitation pendant 30 minutes. Peser Phase C, mélanger jusqu'à obtenir un mélange homogène. Ajouter Phase C à Phase A+B sous agitation. Ajouter Phase au mélange précédent. Ajouter Phase E au mélange précédent sous agitation. Bien homogénéiser. Peser Phase F, mélanger et ajouter au mélange précédent. Mélanger de manière insistante.

### Exemple 13 : Crème de jour

| **Noms INCI** | **% en masse** |
|---|---|
| **Phase A** | |
| Eau | Qsp 100 |
| Carbomère | 0,2 |

| **Phase B** | |
|---|---|
| Butylène Glycol | 2,00 |
| Phenoxyethanol | 1,30 |

| **Phase C** | |
|---|---|
| Glyceryl stéarate & PEG 100 stéarate | 1,00 |
| Caprylic/capric Triglycérides | 4,00 |

| **Phase D** | |
|---|---|
| Acrylates/C 10-30 Alkyl Acrylates cross polymer | 0,20 |
| PPG-3 Benzyl Ether Myristate | 1,00 |
| Dimethicone | 1,00 |

| **Phase E** | |
|---|---|
| Sorbate | 0,10 |

| **Phase F** | |
|---|---|
| Hydroxyde de sodium | 0,40 |
| Eau | 4,00 |

| **Phase G** | |
|---|---|
| Ingrédient actif selon l'invention (selon exemple 1 ou 2 ou 3) | 2,00 |
| Phase H | |
| Fragrance | 0,10 |

Mode opératoire : Phase A : Verser Ultrez 10 dans l'eau et laisser gonfler pendant 30 minutes. Peser Phase B et laisser fondre à 60 °C. Chauffer Phase A dans un bain d'eau à 75°C. Peser Phase C et chauffer à 75°C dans un bain d'eau. Sous agitation (Starvo v=500 t/min), ajouter Phase C à Phase A. Ajouter arbitrairement Phase B et Phase D dans Phase A+B puis y ajouter Phase E. Bien homogénéiser. Refroidir à 45°C et ajouter Phase F. A 35°C, ajouter Phase G, bien homogénéiser. Ajouter Phase H, bien homogénéiser.

### Exemple 14 : Fluide pour le corps.

| **Noms INCI** | **% en masse** |
|---|---|
| **Phase A** | |
| Eau | 5,00 |
| Carbomère | 0,30 |

| **Phase B** | |
|---|---|
| Eau | Qsp 100 |
| Hydroxyethyl cellulose | 0,40 |

| **Phase C** | |
|---|---|
| Butylène Glycol | 3,00 |
| Phenoxyethanol | 1,30 |

| **Phase D** | |
|---|---|
| C12-C15 Alkyl Benzoate | 2,00 |
| Caprylic/capric Triglycéride | 3,00 |
| Polysorbate 20 | 1,00 |
| PPG-3 Benzyl Ether Myristate | 1,00 |
| Acrylates/C10-30 Alkyl Acrylates cross polymer | 0,20 |

| **Phase E** | |
|---|---|
| Sorbate | 0,10 |

| **Phase F** | |
|---|---|
| Hydroxyde de sodium | 0,50 |
| Eau | 5,00 |

| **Phase G** | |
|---|---|
| Ingrédient actif selon l'invention (selon exemple 1 ou 2 ou 3) | 2,00 |

| **Phase H** | |
|---|---|
| Fragrance | 0,10 |

Mode opératoire : Phase A : Verser Ultrez 10 dans l'eau et laisser gonfler pendant 30 minutes. Sous agitation à hélice (300 t/min), verser Phase B et laisser gonfler pendant 1h. Ajouter Phase A dans Phase B sous agitation à hélice (300 t/min), bien homogénéiser. Peser et mettre sous agitation Phase C, peser Phase D et mélanger. Ajouter Phase C à Phase A+B. Ajouter Phase D à Phase A+B+C. Bien homogénéiser. Ajouter Phase E puis Phase F et G et enfin Phase H. pH = 6.2.

### Exemple 15 : Crème de nuit

| **Noms INCI** | **% en masse** |
|---|---|
| **Phase A** | |
| Water | 5,00 |
| Carbomer | 0,30 |

| **Phase B** | |
|---|---|
| Eau | Qsp 100 |
| Hydroxyethyl cellulose | 0,40 |

| **Phase C** | |
|---|---|
| Butylène Glycol | 3,00 |
| Phenoxyethanol | 1,30 |

| **Phase D** | |
|---|---|
| PPG-3 Benzyl Ether Myristate | 1,00 |
| Acrylates/C10-30 Alkyl Acrylates cross polymer | 0,20 |

| **Phase E** | |
|---|---|
| Sorbate | 0,10 |

| **Phase F** | |
|---|---|
| Hydroxyde de sodium | 0,50 |
| Eau | 5,00 |

| **Phase G** | |
|---|---|
| Ingrédient actif selon l'invention (selon exemple 1 ou 2 ou 3) | 2,00 |

| **Phase H** | |
|---|---|
| Fragrance | 0,10 |

Mode opératoire : Phase A : Verser Ultrez 10 dans l'eau et laisser gonfler pendant 30 minutes. Sous agitation à hélice (300 t/min), verser Phase B et laisser gonfler pendant 1h. Ajouter Phase A dans Phase B sous agitation à hélice (300 t/min), bien homogénéiser. Peser et mettre sous agitation Phase C, peser Phase D et mélanger. Ajouter Phase C à Phase A+B sous agitation à couteau (300 t/min). Ajouter Phase D à Phase A+B+C. Bien homogénéiser. Ajouter Phase E puis Phase F et G et enfin Phase H. Bien homogénéiser.

### Exemple 16: Crème anti-vergeture

| **Noms INCI** | **% en masse** |
|---|---|
| **Phase A** | |
| Eau | Qsp 100 |
| Carbomère | 0,40 |

| **Phase B** | |
|---|---|
| Glycérine | 5,00 |
| Phenoxyethanol (and) Mixed Parabens | 0,80 |

| **Phase C** | |
|---|---|
| Ethylhexyl Palmitate | 4,00 |
| Cetearyl alocholCroda | 0,50 |
| Myristyl Lactate | 0,30 |
| Polysorbate 20 | 1,00 |

| **Phase D** | |
|---|---|
| Acrylates/C10-30 Alkyl Acrylates cross polymer | 0,20 |
| Cyclomethicone | 2,00 |

| **Phase E** | |
|---|---|
| Sorbate de potassium | 0,10 |

| **Phase F** | |
|---|---|
| Hydroxyde de sodium | 0,60 |
| Eau | 6,00 |

| **Phase G** | |
|---|---|
| Ingrédient actif selon l'invention (selon exemple 1 ou 2 ou 3) | 1,00 |

| **Phase H** | |
|---|---|
| Fragrance | 0,10 |

Mode opératoire :
Phase A : Disperser Ultrez 10 dans l'eau et laisser gonfler pendant 20 minutes. Mélanger Phase B et chauffer à 60°C jusqu'à dilution complète. Ajouter Phase B à Phase A sous agitation. Chauffer Phase A+B. Peser Phase C et chauffer à 75°C. Ajouter Phase C à Phase A+B sous agitation. Homogénéiser avec précaution puis ajouter Phase D. Ajouter Phase E. Neutraliser avec Phase F à 50 °C. Ajouter Phase G et H à 35°C et ajuster le pH à 6,3 avec NaOH.

### Exemple 17 : Lotion pour cheveux

| **Noms INCI** | **% en masse** |
|---|---|
| **Phase A** | |
| Chlorure de Cetrimonium | 1,00 |
| Acide citrique | 0,22 |
| Citrate Trisodique | 1,20 |
| Sorbate | 0,10 |
| Eau | Qsp 100 |

| **Phase B** | |
|---|---|
| Methyl Paraben | 0,20 |
| PPG 5 Ceteth 20 | 2,00 |

| **Phase C** | |
|---|---|
| Ingrédient actif selon l'invention (selon exemple 1 ou 2 ou 3) | 1,00 |

| **Phase D** | |
|---|---|
| Polysorbate 20 | 1,00 |
| Fragrance | 0,10 |

### Exemple 18 : Maquillage hydratant.

| **Noms INCI** | **% en masse** |
|---|---|
| **Phase A** | |
| Eau | Qsp 100 |
| Hydroxide de potassium | 1,3 |
| Polysorbate 80 | 0,1 |

| **Phase B** | |
|---|---|
| Titanium dioxide | 6,00 |
| Talc | 3,05 |
| Yellow iron oxide | 1,8 |
| Red iron oxide | 1,00 |
| Black iron oxide | 0,15 |
| Phase C | |
| Propylene glycol | 4,00 |
| Magnésium Aluminium Silicate | 1,00 |
| Phase D | |
| Propylene glycol | 2,00 |
| Sodium Carboxymethylcellulose | 0,12 |
| Phase E | |
| Di-PPG-3 Myristyl Ether Adipate | 12,00 |
| Isostearyl Neopentanoate | 4,00 |
| Cetearyl Alcohol, Ceteth-20 Phospahte, Dicetyl Phosphate | 3,00 |
| Steareth-10 | 2,00 |
| Cetyl alcohol | 0,62 |
| Steareth-2 | 0,5 |
| Phase F | |
| Ingrédient actif selon l'invention (selon exemple 1 ou 2 ou 3) | 3,00 |

### Exemple 19 : Baume à lèvre.

| **Noms INCI** | **% en masse** |
|---|---|
| **Phase A** | |
| Eau | Qsp 100 |
| Sorbate de potassium | 0,10 |
| Sulfate de magnésium | 0,70 |

| **Phase B** | |
|---|---|
| Cetyl Dimethicone Copolyol | 3,00 |
| Methyl Paraben | 1,00 |
| Tribehenin | 0,30 |
| PPG-3 BEnzyl Ether Myristate | 2,00 |
| Argania spinoa Kernel Oil | 19,00 |

| **Phase C** | |
|---|---|
| Ingrédient actif selon l'invention (selon exemple 1 ou 2 ou 3) | 0,50 |

| **Phase D** | |
|---|---|
| Fragrance | 0,10 |

Mode opératoire : Chauffer Phase A à 85°C. Mélanger Phase B et chauffer à 85°C. Ajouter doucement Phase A à Phase B sous agitation (Staro v=3000 t/min puis 1200 t/min). Ajouter Phase C préchauffée à 80°C, homogénéiser. Ajouter Phase D à 35°C. Verser.

### Exemple 20 : Spray protecteur cheveux

| **Noms INCI** | **% en masse** |
|---|---|
| **Phase A** | |
| Eau | Qsp 100 |
| Ethanol | 10,00 |
| Polysorbate 20 | 0,40 |
| Chlorure de Cetrinium | 1,00 |

| **Phase B** | |
|---|---|
| Butylène glycol (and) Helianthus Annus Seed Extract | 5,00 |
| Conservateur | Qs |

| **Phase C** | |
|---|---|
| Ingrédient actif selon l'invention (selon exemple 1 ou 2 ou 3) | 3,00 |

| **Phase D** | |
|---|---|
| Eau | 0,50 |
| Hydroxyde de sodium | 0,05 |

Mode opératoire : Peser et mélanger la Phase A avec un agitateur à couteau v = 300 t/min. Ajouter la Phase B, mélanger puis ajouter la Phase C. Ajuster le pH entre 5,0 et 5,5 avec la Phase D.

### Exemple 21 : Tests démontrant l'activité anti-âge et raieunissante de l'extrait d'Uchuva

Dans les figures accompagnant les exemples 21 à 23, l'extrait d'Uchuva est abrégé EUc. Dans tous ces exemples 21 à 23, l'extrait d'Uchuva provient du procédé d'extraction décrit dans l'exemple 3 présentant 99,9% de pureté chromatographique en sucrose-esters.

Le derme fournit à l'épiderme un support solide, c'est également son élément nourricier. Il est principalement constitué de fibroblastes et d'une matrice extracellulaire composée majoritairement de collagènes, d'élastine et d'une substance, dite substance fondamentale. Ces composants sont synthétisés par les fibroblastes. La cohésion entre l'épiderme et le derme est assurée par la jonction dermo-épidermique.

Les collagènes sont les protéines majoritaires des matrices extracellulaires de la peau. A ce jour, 20 types de collagènes sont identifiés et notés de 1 à XX. Les collagènes majoritairement présents dans l'ensemble du derme sont les collagènes de type I et III qui forment la matrice extracellulaire de l'ensemble du derme (ce sont ces collagènes qui constituent 70-80 % du poids sec du derme). Avec le vieillissement, le derme s'amincit et des rides apparaissent à la surface de la peau. En conséquence, compte tenu du rôle important du collagène au niveau de l'intégrité de la peau et de sa résistance aux agressions externes de type mécaniques, la stimulation de la synthèse de ces collagènes, et en particulier du collagène de type I et III, apparaît comme un moyen efficace pour pallier les signes du vieillissement cutané (revue par Tzaphlidou M., Micron 35 (2004) 173-177).

### - Cibles Collagène 1 et III

Le Collagène I est le Collagène majoritaire qui apporte à la peau sa résistance mécanique.

Cette protéine représente 90 % du collagène d'un vertébré. Il constitue la trame de l'os (à comparer aux armatures du béton armé), et plus généralement des tissus conjonctifs banals. Il se trouve dans les os, la peau, les tendons, la cornée et les organes internes.

### 1 - Etude sur cellules jeunes versus cellules vieillies

L'étude qui suit a permis d'étudier l'effet de l'extrait d'Uchuva sur l'expression du collagène I, constituant essentiel de la matrice extracellulaire du derme.

### - Méthode

Les NHDF (Normal Human Dermal Fibroblasts) jeunes ou vieillis par sénescence réplicative ont été ensemencés en plaque 96 puits et incubées 24h à 37°C, 5%CO2.

Les cellules ont été traitées 24h en présence des produits d'essai.

Les cellules ont ensuite été fixées avec de la formaline et l'expression des protéines a été détectée par immunofluorescence.

Les marquages fluorescents ont été imagés et quantifiés par microscopie automatisée (Arrayscan CellomicsTM). La fluorescence a été quantifiée par la bioapplication Compartimental Analysis.

### - Evaluation préalable de la cytotoxicité

Le composé a été mis en contact avec les cellules pendant 24 heures. Durant les 3 dernières heures du test calorimétrique prêt à l'emploi WST1 de Roche^{®} a été introduit dans le milieu.

Ce réactif contient des sels de tetrazolium, un indicateur violet. Ce réactif est clivé en formazan, un indicateur jaune, par les cellules métaboliquement actives. Le niveau de coloration jaune est donc proportionnel au nombre de cellules vivantes. La mesure de l'absorbance se fait à 450nm.

Le test considère qu'une valeur inférieure à 90% du témoin indique une cytotoxicité du produit éventuelle (symbolisée par une ligne verte dans les graphes). Cela peut aussi indiquer que l'on diminue l'activité métabolique des cellules. Une valeur inférieure à 75% indique une cytotoxicité significative (symbolisée par une ligne rouge dans les graphes)
Par ailleurs les cellules ont été observées au microscope pour observer et comparer leur physionomie.

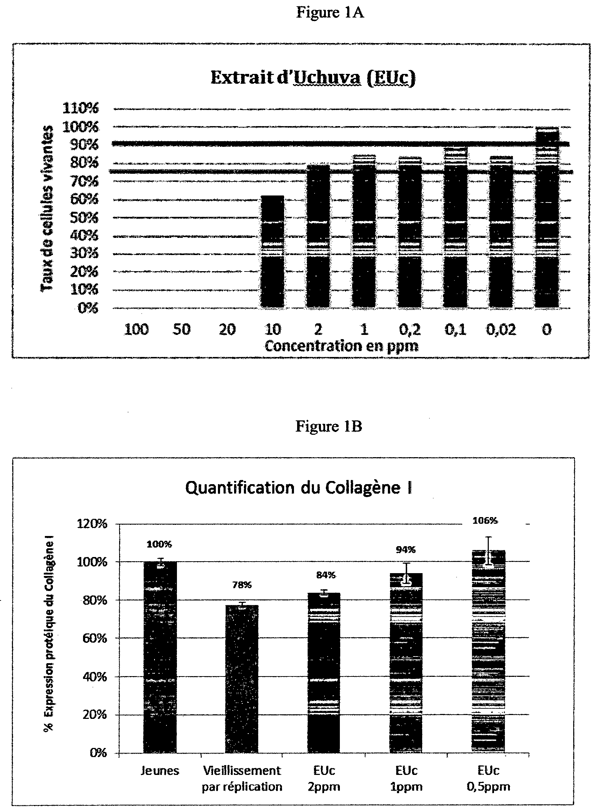

Les résultats sont donnés dans la figure 1A.

L'extrait d'Uchuva est cytotoxique aux plus fortes concentrations testées de 100 à 20 ppm et devient moins cytotoxique à partir de 2ppm.

### Concentrations retenues pour être testées :

L'Extrait d'Uchuva est testé aux concentrations suivantes 2 - 1 - 0,5 ppm.

Les résultats sont donnés dans la figure 1B.

L'extrait d'Uchuva permet de rétablir l'expression de Collagène I au niveau des cellules jeunes. Dans les cellules jeunes l'expression du Collagène I n'est pas homogène, certaines cellules présentent un marquage fort et d'autres un marquage plus faible. Au sein des cellules vieillies par réplication, l'expression réactivée par l'extrait d'Uchuva est plus homogène dans les cellules traitées.

Le fait que la concentration la plus basse soit active peut s'expliquer par le fait que les concentrations les plus fortes présentent une légère cytotoxicité qui affecte la synthèse protéique.

### - Conclusion

L'extrait d'Uchuva, est un ingrédient qui agit sur le collagène I, cible du vieillissement cutané, en rétablissant l'expression protéique à hauteur de celle observée dans les cellules jeunes.

### 2 - Evaluation de l'effet de l'extrait d'Uchuva sur la production de Collagène 1 par les fibroblastes

### Matériels et méthodes utilisées

### - Système d'essai

Désignation : Modèle Fibroblastes humains (NHDF) en culture utilisés entre les passages 3 à 6.
Milieu de culture : DMEM glucose 4,5 g/L + 1% NEAA + 10% SVF
Conditions de culture : 37°C, 5% de CO2

### - Méthode de culture et traitement des fibroblastes

### - Evaluation de la cytotoxicité par le test du rouge neutre

Les cellules NHDF sont ensemencées à J0, en plaque 96 puits, à raison de 4×10³ cellules par puits. Elles sont traitées à J1 pendant 48 heures de culture en milieu complet.

Les concentrations de l'actif à tester sont exprimées en % de l'extrait. La solution mère du produit à tester a été préparée dans du diméthylsulfoxyde (DMSO) à la concentration de 20% (p/v). La première dilution a été réalisée au 1/1000^{e}. Les dilutions suivantes ont été réalisées au ½ en maintenant la concentration de DMSO finale à 0,1%.

Le test RN est réalisé à J3: le milieu est éliminé et les cellules sont rincées par 250 µL de PBS préchauffé à 37 °C. 200 µl de rouge neutre sont ajoutés aux cellules. Après 3 heures d'incubation à 37°C, 5% de CO2, le milieu contenant le rouge neutre est éliminé puis les cellules sont rincées avec 2 × 200 µl de PBS pré-chauffé à 37°C. 100 µl de solution de révélation (éthanol 50% - acide acétique 1%) sont ajoutés et les cellules sont incubées à température ambiante sous agitation et à l'abri de la lumière pendant 45 minutes.

La DO₅₄₀ₙₘ est mesurée après homogénéisation.

NB : Préparation de la solution de rouge neutre : la solution mère de rouge neutre est préparée à 0,4% dans l'eau Ultra Pure (cette solution se conserve à 4°C pendant 15 jours), elle est diluée extemporanément au 1/80ème dans le milieu de culture complet puis centrifugée 10 minutes à 3000 tours par minute (tpm) avant utilisation.

### -Evaluation de l'activité métabolique par le test MTT (Méthyl Thiazol Tétrazolium)

Les cellules NHDF sont ensemencées à J0, en plaque 96 puits, à raison de 4×10³ cellules par puits. Elles sont traitées à J1 pendant 48 heures de culture en milieu complet.

Les concentrations de l'actif à tester sont exprimées en % de l'extrait. La solution mère de l'extrait a été préparée dans du DMSO à la concentration 20% (p/v). La première dilution a été réalisée au 1/1000^{e}. Les dilutions suivantes ont été réalisées au ½ en maintenant la concentration de DMSO finale à 0,1%.

Le test MTT est réalisé à J3 : le milieu est remplacé par 100 µL de MTT à 0,5 g/L dans du milieu complet. Après 3 heures d'incubation à 37°C, 5% de CO2, la solution de MTT est remplacée par 100 µL de DMSO. La DO540nm est mesurée après homogénéisation (condition de dosage comme précisé pour le test précédent au rouge neutre).

### - Produit testé et mode de préparation

Dénomination du produit à tester : Extrait d'Uchuva
A l'issue des 2 premiers tests donnés ci-dessus, 3 concentrations du produit à tester ont été sélectionnées pour la suite des tests à réaliser : 8.10⁻⁵%(0,8 ppm), 16.10⁻⁵% (1,6 ppm) et 32. 10⁻⁵% (3,2 ppm)

### - Dosage du collagène I dans les surnageants de culture par méthode ELISA

Les cellules sont ensemencées à J0, sur plaques 6 puits, à raison de 25×10³ cellules par puits. Elles sont traitées à J1 pendant 48 heures de culture en milieu complet.

Les concentrations de l'actif à tester sont exprimées en % de l'extrait. La solution mère de l'extrait a été préparée dans du DMSO à la concentration de 20% (p/v). La première dilution a été réalisée au 1/1000^{e}. Les dilutions suivantes ont été réalisées en maintenant la concentration de DMSO finale à 0,016% (correspondant à la concentration de DMSO à la plus forte concentration de l'extrait).

Après 48h, les surnageants de culture sont récupérés et stockés à -80°C.

Le dosage collagène I est réalisé selon les instructions du fournisseur du kit ELISA (Tecomedical).

Effet de l'extrait d'Uchuva sur la production de collagène I par des fibroblastes en culture 48h:

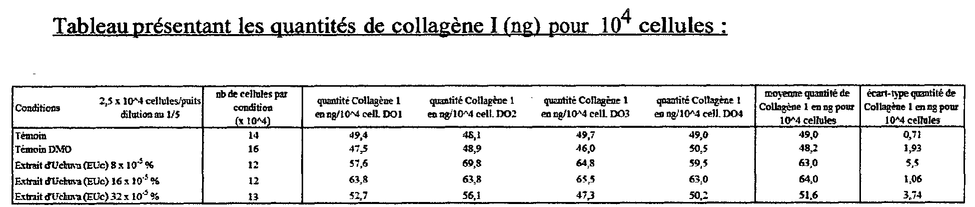

Ces résultats permettent de constater une augmentation significative de la production de Collagène I de 29% et 30% par les fibroblastes traités pendant 48 h par l'extrait d'Uchuva aux concentrations les plus faibles (respectivement 8.10⁻⁵% et 16.10⁻⁵%) par rapport au contrôle.

A la concentration la plus forte (32. 10⁻⁵%), la production de Collagène I par les fibroblastes diminue à un niveau proche des conditions contrôles. Cette diminution peut être expliquée par un début de toxicité cellulaire à cette plus forte concentration.

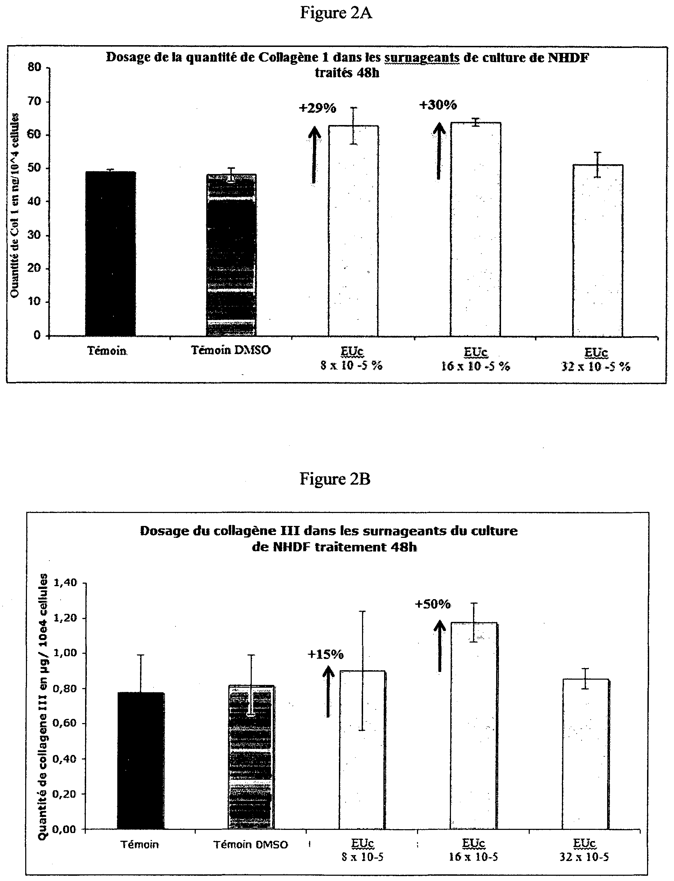

Les résultats sont présentés dans la figure 2A.

### 3- Evaluation de l'effet de l'extrait d'Uchuva sur la production de Collagène III par les fibroblastes

Les cellules sont ensemencées à J0, sur plaques 6 puits, à raison de 25×10³ cellules par puits. Elles sont traitées à J1 pendant 48 heures de culture en milieu complet.

Les concentrations de l'actif à tester sont exprimées en % de l'extrait. La solution mère de l'extrait a été préparée dans du DMSO à la concentration de 20% (p/v). La première dilution a été réalisée au 1/1000^{e}. Les dilutions suivantes ont été réalisées en maintenant la concentration de DMSO finale à 0,016% (correspondant à la concentration de DMSO à la plus forte concentration de l'extrait) :

| | | |
|---|---|---|
| **EUc 8 x 10 -5 %** | **EUe 16 x 10 -5 %** | **EUc 32 x 10 -5 %** |

Après 48h, les surnageants de culture sont récupérés et stockés à -80°C.

Le dosage collagène III est réalisé selon les instructions du fournisseur du kit ELISA (SunRed).

Effet de l'extrait d'Uchuva sur la production de collagène III par des fibroblastes en culture 48h:

**Tableau suivant présentant les quantités de collagène III (µg) pour 10⁴ cellules :**

| **Quantité de Collagène III en µg/10⁴ cellules** | MOYENNE | ET |
|---|---|---|
| Témoin | 0,78 | 0,21 |
| Témoin DMO | 0,82 | 0,17 |
| Extrait d'Uchuva (EUc) 8 x 10⁻⁵ % | 0,9 | 0,34 |
| Extrait d'Uchuva (EUc) 16 x 10⁻⁵ % | 1,17 | 0,11 |
| Extrait d'Uchuva (EUc) 32 x 10⁻⁵ % | 0,86 | 0,06 |

L'analyse de ces résultats met en évidence une augmentation de la concentration dépendante de la production de Collagène III induite par l'extrait d'Uchuva. La production est maximale à la concentration de 16.10⁻⁵% (+50% par rapport aux contrôles).

A la concentration de l'extrait la plus forte (32.10⁻⁵%), la production de Collagène III par les fibroblastes diminue à un niveau proche de celle de la condition contrôle comme dans le cas de la production de Collagène I. Cette observation confirme la présence d'une toxicité cellulaire à ce niveau de concentration.

Les résultats sont présentés dans la figure 2B.

### 4 - Evaluation de l'effet de l'extrait d'Uchuva sur la cible Collagène IV

Le collagène est le constituant majoritaire de la MEC (environ 70%) synthétisé par les fibroblastes. Il existe les collagènes fibrillaires (I, II, III, V, XI) et non fibrillaires (IX, XII, XIV, XVI). Les fibres de collagènes sont flexibles et elles résistent aux forces de tension dans les tissus. Elles forment des faisceaux ondulés de longueur et d'épaisseur variable. Le collagène de type IV est particulier, il forme le « réseau primaire » des membranes basales (lamina densa). Il est impliqué dans l'adhésion cellulaire, la prolifération, la migration et dans l'angiogenèse (mélanome). Il possède un site d'interaction avec le récepteur aux intégrines. Il est situé au point de contact entre la lame basale et la MEC (S Pasco et al., Cancer Detection and Prevention, 29,260, 2005). Le collagène IV, avec les laminines 1, forme un réseau qui constitue la charpente de la membrane basale afin de réaliser un maintien structural et fonctionnel des tissus.

Les deux concentrations de 4.10⁻⁵% et 8.10⁻⁵% de l'extrait d'Uchuva ont été testées.

### Evaluation de la quantité de protéines de la matrice extracellulaire produites par les fibroblastes durant 48h

### - Détection de l'expression du collagène IV par immunocytologie

Les cellules sont ensemencées à J0, sur lames Millicell 8 puits, à raison de 4.10³ cellules par ml dans 400 µl de milieu complet. Elles sont traitées à J1 pendant 48 heures de culture en milieu complet. Les concentrations de l'actif à tester sont exprimées en % de l'extrait. La solution mère de l'extrait a été préparée dans du DMSO à la concentration de 20% (p/v). La première dilution a été réalisée au 1/1000^{e}. Les dilutions suivantes ont été réalisées en maintenant la concentration de DMSO finale à 0,1%. Après 48h, les cellules sont fixées par un mélange alcool/acide. Les cellules sont ensuite marquées à l'aide d'un anticorps anti-collagène IV (Rockland) dilué dans du PBS + BSA/tween au 1/50 pendant 1 h. Les lames sont ensuite rincées 2 fois par du PBS. La révélation du marquage spécifique se fait à l'aide d'un anticorps secondaire couplé FITC (Santa-Cruz) dilué dans du PBS + BSA/tween au 1/100 pendant 1 heure. Les lames sont ensuite rincées 2 fois par du PBS et montées entre lame et lamelle à l'aide du liquide de montage RotiMount Fluocare + DAPI (Roth).

Des photos dans le vert et dans le bleu ont été réalisées à l'aide d'une camera DP72 (Olympus, Japan) couplé à un microscope à épifluorescence BX-60 (Olympus, Japan).

Les photos ont été observées par 2 lecteurs et un scoring de l'intensité de fluorescence a été réalisé sur une échelle linéaire allant de 0 (pas de marquage) à 4 (très fort marquage).

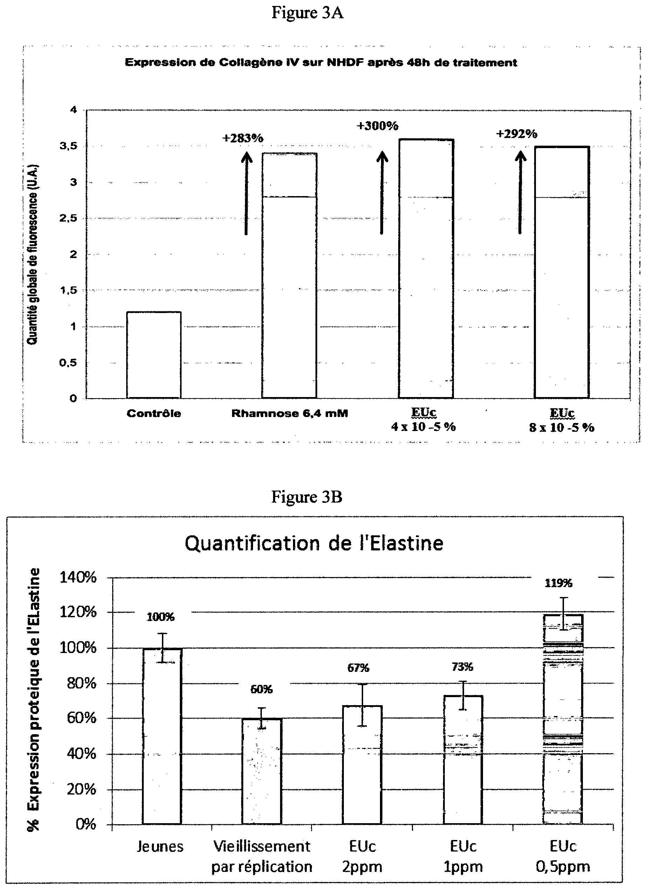

En parallèle, une concentration de rhamnose a été intégrée à cet essai en tant que contrôle positif pour l'expression du Collagène IV. En effet, le rhamnose et les polysaccharides contenant du rhamnose sont décrits pour induire la synthèse des collagènes dans les fibroblastes²². Les résultats sont présentés dans la figure 3A.

L'analyse des résultats met en évidence une forte augmentation de l'expression du Collagène IV (environ +300%) dans les fibroblastes traités pendant 48 h par l'extrait d'UCHUVA et ceci quelque soit la concentration utilisée. L'amplitude de cette augmentation d'expression du Collagène IV est comparable à celle obtenue avec le rhamnose.

### 5 - Evaluation de l'effet de l'extrait d'Uchuva sur la cible Elastine

L'élastine est une glycoprotéine structurale (comme la laminine et la fibronectine) entrant dans la composition de la MEC. L'élastine est une protéine de la famille des protéines fibreuses de type structural. Sécrétée par les fibroblastes essentiellement durant la période de croissance, elle possède des propriétés élastiques. Sa synthèse diminue avec l'âge et l'élastine se trouve remplacée par du collagène inextensible. Les vergetures sont un exemple visible de ce processus, qui est lié à des contraintes mécaniques. Le vieillissement cutané en est un deuxième exemple.

### Dans la matrice extracellulaire :

L'élastine est synthétisée et sécrétée dans l'espace extracellulaire par les fibroblastes d'abord en proélastine, puis en tropoélastine. L'élastine est la composante majeure (jusqu'à 90 %) des fibres élastiques auxquelles s'ajoute la fibrilline. Donc, le collagène, associé à l'élastine et la fibrilline qui forment les fibres élastiques, par des liaisons transversales covalentes, sont les principaux constituants de la matrice extracellulaire. La production totale d'élastine s'arrête autour de la puberté. Après quoi, la quantité d'élastine disponible diminuera avec le temps.

### Dégradation :

La dégradation de l'élastine est liée à l'action de l'élastase, une enzyme sécrétée par les fibroblastes. L'action enzymatique de l'élastase est inhibée par l'α1-antitrypsine. L'inhibition de la dégradation crée un équilibre augmentant la stabilité de l'élastine.

### Rôle :

Des traits distinctifs caractérisent l'élastine : l'élastine permet aux cellules de se lier et permet aux tissus biologiques de se former. Ainsi, le bon fonctionnement de la peau, des poumons, des vaisseaux sanguins, des tissus conjonctifs, de certains tendons et cartilages est étroitement lié aux caractéristiques de l'élastine. Comme son nom l'indique, l'élastine est élastique. À diamètre égal, elle est 5 fois plus élastique qu'un élastique. Elle peut s'étirer jusqu'à 150% de sa longueur au repos avant de se briser. Ainsi, elle permet aux tissus de s'étirer et de retrouver leur état initial après l'étirement, ce qui leur donne de la souplesse.

### Le derme :

L'élastine se retrouve dans le derme de la peau, celui-ci agissant en tant que soutien. Au cours du vieillissement, par exemple, la perte d'élasticité et de tonicité du derme qui ne peut plus s'opposer aux effets de contraction des muscles sous-jacents donne lieu à l'apparition des rides. Par ailleurs, l'exposition aux ultraviolets augmente la dégradation de l'élastine.

### Etude sur cellules jeunes versus cellules vieillies

L'étude qui suit a permis d'étudier l'effet de l'extrait d'Uchuva sur l'expression de la protéine élastine.

### Méthode

Les NHDF (Normal Human Dermal Fibroblasts) jeunes ou vieillis par senescence réplicative ont été ensemencés en plaque 96 puits et incubées 24h à 37°C, 5%CO2.

Les cellules ont été traitées 24h en présence des produits d'essai. Les cellules ont ensuite été fixées avec de la formaline et l'expression des protéines a été détectée par immunofluorescence. Les marquages fluorescents ont été imagés et quantifiés par microscopie automatisée (Arrayscan Cellomics^{™}), La fluorescence a été quantifiée par la bioapplication Compartimentai Analysis.

### Modèles cellulaires :

- Jeunes : NHDF de donneur jeune (25 ans)
- Vieillissement par senescence réplicative : NHDF de donneur jeune cultivés sur plusieurs passages jusqu'à atteindre la senescence.

Les résultats sont présentés dans la figure 3B.

L'extrait d'Uchuva permet de rétablir l'expression de l'Elastine légèrement au-dessus du niveau protéique des cellules jeunes.

### Conclusion

Au vu des résultats obtenus, l'extrait d'Uchuva présente une amélioration de l'élasticité in vitro qui pourra être extrapolée in vivo.

### 6- Evaluation de l'effet de l'extrait d'Uchuva sur la cible méthylation de l'ADN

La méthylation est une modification des extrémités N-terminales des histones. Elle peut s'effectuer soit sur des lysines soit sur des arginines et peut se concrétiser par l'ajout d'un, de deux ou de trois groupements méthyles. Selon les résidus méthylés et le nombre de groupements ajoutés elle est associée à une activation ou une répression de la transcription. Longtemps considérée comme statique, la méthylation des histones s'avère être une modification réversible impliquée dans un processus dynamique, bien que plus stable que l'acétylation et la phosphorylation. Un nombre croissant d'histones déméthylases sont identifiés. De manière générale, ce type de modifications est antagoniste à l'acétylation, et la désacétylation des lysines doit précéder leur méthylation. Cet antagonisme entraîne la mise en place d'un certain équilibre dynamique entre les territoires hétérochromatiniens (généralement non exprimables et méthylés sur certains acides aminés clés) et euchromatiniens (généralement exprimables et acétylés). Par exemple, la Lysine 9 de l'histone H3 est connue pour être associée à une répression de la chromatine environnante lorsqu'elle est méthylée. Cette méthylation est reconnue par une protéine, HP1, qui se fixe donc sur H3 méthylée. À son tour, HP1 attire la protéine Suv39, une Histone MethylTransférase, qui pourra méthyler la lysine 9 de l'histone H3 du nucléosome voisin, et ainsi de suite. On voit donc, comment, de proche en proche, les histones H3 seront méthylées et la chromatine sera condensée. Cependant, cette invasion hétérochromatinienne sera stoppée si la lysine 9 de H3 rencontrée est déjà acétylée. Ainsi se met en place un équilibre compétitif entre domaines chromatiniens exprimés et réprimés. Les modifications des queues d'histones jouent le rôle de « marques » épigénétiques qui entraînent le recrutement de différentes classes de protéines, puisque les lysines acétylées ou méthylées sont reconnues par des domaines protéiques différents. De plus, le recrutement de certains facteurs au niveau de la chromatine nécessite l'existence préalable de modifications d'histones et de protéines déjà liées. Le code des histones est donc interprété dans le contexte d'autres facteurs associés à la chromatine et c'est la combinaison d'interaction entre les histones modifiées et d'autres facteurs qui détermine si une protéine est recrutée à la chromatine. Tous les tissus des organismes sont affectés par le vieillissement. Ce processus est lié aux modifications épigénétiques telles que les changements de méthylation au niveau de résidus cytosine spécifiques de l'ADN, tel que décrit dans de nombreuses publications²⁴⁻³¹. Le rôle des modifications épigénétiques sur le vieillissement, l'accumulation de divisions cellulaires et de macromolécules détériorées contribuent à un phénotype âgé. Les évènements environnementaux et aléatoires peuvent de plus modifier ce phénotype par l'intermédiaire de mécanismes épigénétiques tels que la méthylation de l'ADN et la méthylation et l'acétylation des histones. La potentielle réversibilité des modifications épigénétiques fait d'eux des cibles attractives pour le traitement des pathologies liées au vieillissement.

### Etude sur cellules jeunes versus cellules vieillies

Afin de comprendre le mécanisme de réactivation de l'expression des protéines observée, nous avons procédé à une analyse de la méthylation de l'ADN sur cellules vieillies de manière intrinsèque. En effet l'une des signatures de la sénescence cellulaire est l'accumulation de zones méthylées au sein de l'ADN, au niveau des Cytosines contenues dans les dinucléotides CpG. Ceci entraîne l'inactivation de promoteurs et la diminution de l'expression de certains gènes.

### - Méthode

Les NHDF (Normal Human Dermal Fibroblasts) jeunes ou vieillis de manière intrinsèque par réplication ont été ensemencés en plaque 6 puits et incubées à 37°C, 5%CO2 jusqu'à subconfluence. Les cellules ont été traitées 24h en présence des produits d'essai. Les cellules ont ensuite été décollées en présence de trypsine et lysées. L'ADN génomique a été précipité à l'Ethanol. Le taux de méthylation de l'ADN a été dosé par ELISA en utilisant le kit Enzo : 5-Methylcytosine DNA ELISA kit. Les valeurs sont représentées à partir de 50% pour une meilleure visibilité des résultats.

### Modèles cellulaires :

- Jeunes : NHDF de donneur jeune (25 ans)
- Vieillissement par senescence réplicative : NHDF de donneur jeune cultivés sur plusieurs passages jusqu'à atteindre la senescence.

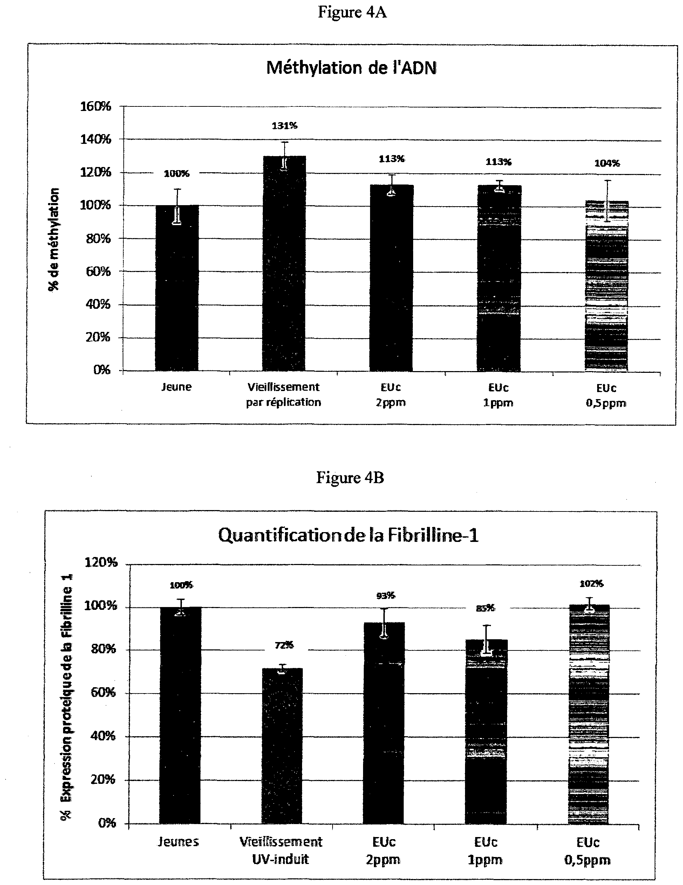

Suite à l'évaluation préalable de sa cytotoxicité, l'extrait d'Uchuva est testé aux concentrations suivantes 2 - 1 - 0,5 ppm.
- Résultats : Les résultats sont présentés dans la figure 4A.

### Conclusion

Au vu des résultats obtenus, l'extrait d'Uchuva permet de réduire le taux de méthylation aux trois concentrations testées par rapport au modèle de peau vieillie.

### 7 - Evaluation de l'effet de l'extrait d'Uchuva sur la cible Fibrilline-1

La Fibrilline 1 est une protéine qui constitue les microfibrilles, lesquelles sont associées aux fibres élastiques et participent à leur assemblage.

### Etude sur cellules jeunes versus cellules vieillies

### Méthode

Les NHDF (Normal Human Dermal Fibroblasts) jeunes ont été ensemencés en plaque 96 puits et incubées 24h à 37°C, 5%CO2. Les cellules destinées au vieillissement extrinsèque ont été irradiées 3 fois aux UVA avec 24h entre chaque irradiation et 1 fois aux UVB. Les cellules ont été traitées en présence des produits d'essai entre chaque irradiation. Les cellules ont ensuite été fixées avec de la formaline et l'expression des protéines a été détectée par immunofluorescence. Les marquages fluorescents ont été imagés et quantifiés par microscopie automatisée (Arrayscan Cellomics^{™}). La fluorescence a été quantifiée par la bioapplication Compartimental Analysis.

### Modèles cellulaires :

- Jeunes : NHDF de donneur jeune (25 ans)
- Vieillissement UVA/B-induit : NHDF de donneur jeune, irradiés aux UVA et aux UVB

### Produits testés

L'extrait d'Uchuva est testé aux concentrations suivantes :2 - 1 - 0,5 ppm suite à l'évaluation préalable de sa toxicité.

### Résultats

Les résultats sont présentés dans la figure 4B.

L'extrait d'Uchuva à 0.5 ppm permet de rétablir l'expression de la Fibrilline 1 à un niveau équivalent voire légèrement supérieur à celui des cellules jeunes.

### - Conclusion générale sur l'activité anti-âge et rajeunissante

Au cours du vieillissement les fibres de collagène et les fibres élastiques sont altérées du fait d'une synthèse réduite et d'une dégradation accrue. Pour démontrer que l'extrait d'Uchuva agit sur le rajeunissement cellulaire, l'expression des protéines impliquées dans la structure de ces fibres au sein de cellules vieillies de manière intrinsèque (senescence réplicative) dans le cas du Collagène I et de l'Elastine, ou extrinsèque par irradiations UV répétées (photo vieillissement) dans le cas de la Fibrilline 1 ont été quantifiées.

L'extrait d'Uchuva permet de rétablir l'expression des 3 marqueurs.

Dans ces études, l'extrait d'Uchuva rétablit en totalité l'expression des protéines en comparaison aux témoins jeunes. C'est tout l'intérêt des effets observés, il n'y a pas de surexpression aberrante, l'extrait d'Uchuva respecte l'équilibre biologique de la peau.

Parallèlement l'extrait d'Uchuva augmente aussi fortement l'expression du Collagène IV fibroblastique par rapport à des cellules non traitées, à la manière du Rhamnose reconnu pour cette vertue. La quantité de Collagène III se trouve aussi accrue lors d'un traitement des fibroblastes par l'extrait d'Uchuva. Ces résultats viennent renforcer son efficacité anti-âge au niveau structural et redensifiant.

Dans le cadre de l'analyse de la méthylation de l'ADN l'extrait d'Uchuva présente des résultats homogènes dans cette expérience, il diminue la méthylation aux 3 concentrations testées. Cet effet donne une information sur le mode d'action de ce produit permettant de réactiver l'expression de protéines de la matrice extracellulaire, lui conférant son effet rajeunissant.

L'extrait d'Uchuva est par conséquent un ingrédient ou principe actif présentant une activité biologique anti-âge, antivieillissement et rajeunissant. Il agit de manière cohérente sur 3 cibles du vieillissement cutané en rétablissant l'expression protéique à hauteur de celle observée dans les cellules jeunes. Il présente également une activité novatrice sur l'épigénétique comme le démontre son action sur la méthylation de l'ADN au sein de cellules matures. Enfin, il démontre une action anti-âge en profondeur par son efficacité sur la synthèse des protéines de la matrice extracellulaire.

### Exemple 22 : Tests démontrant le pouvoir hydratant et structurant de l'épiderme de la peau par l'extrait d'Uchuva

La filaggrine est synthétisée dans le stratum granulosum sous forme d'un précurseur, la profilaggrine (répétition de 10-12 monomères de filaggrine et de la protéine de régulation S-100 NH2 terminale). La détection de cette protéine est associée à un état de différenciation terminal de l'épiderme car elle apparaît après déphosphorylation et protéolyse de la profilaggrine qui a lieu durant la différenciation terminale.
1) La filaggrine participe à l'agrégation de la kératine durant la formation des paquets de macrofibrilles,
2) Les monomères de filaggrine sont des composants de la couche comifiée (CE ; enveloppe qui est déposée à la surface interne de la membrane plasmique des cornéocytes: participe aux propriétés imperméables de la peau avec la matrice lipidique intercornéocytaire),
3) Les monomères sont complètements dégradés sur les couches les plus supérieures du stratum corneum pour produire une mixture d'aminoacides hygroscopiques (NMFs ou Naturel Moisturizing Factors) qui sont importants pour maintenir une hydratation de l'épiderme³².

Dans des situations pathologiques où l'on observe une xérose ou sécheresse de la peau (dermatite atopique, ichtyose), une diminution de la profilaggrine et/ou de la filaggrine a été détectée³³.

Selon Proksch et al³⁴, le suivi de l'expression de la filaggrine est un bon moyen de suivre la fonction barrière et/ou le niveau d'hydratation du Stratum Corneum. C'est le seul réel marqueur qui a du poids comme marqueur d'hydratation des couches supérieures dans les revues de dermatologie. C'est néanmoins en clinique un marqueur qui passe après la mesure du TEWL (TransEpidemal Water Loss).

L'involucrine, comme la filaggrine et la loricrine est un marqueur de différenciation terminale. C'est une protéine de l'enveloppe comifiée (CE) qui est la cible des transglutaminases (TGM) et particulièrement de la TGM1. L'involucrine est oligomérisée par la TGM1 durant les étapes initiales de la formation de l'enveloppe comifiée et sur des étapes plus tardives de la formation de la CE, l'involucrine est liée aux céramides³⁵. Le marquage est localisé dans l'épiderme à partir des couches supérieures de la couche spineuse jusque dans la couche granuleuse et les premières couches du SC.

### - Effet du traitement topique de l'extrait d'Uchuva sur explants de peau humaine

### -Matériel et méthode :

Désignation : Explant de peau mammaire humaine provenant d'une femme de type caucasien âgée de 51 ans (Biopredic - 35000 RENNES).
Nombre : Réalisation de 9 punchs de peau dégraissée d'un diamètre de 1 cm
Conditions de culture : 37°C, 5% de CO₂
Milieu de culture : Milieu DMEM 4,5 g/L de glucose, supplémenté et avec antibiotiques (Pénicilline-streptomycine-Amphotéricine)

### - Produits testés

L'extrait d'Uchuva obtenu selon l'exemple 3 est dilué à 0,05% et 0,01% (p/v) dans de l'huile de paraffine. Le solvant TRIGLYCERIDES C8C10 55/45 (Code EC-09-271) est utilisé.

### - Méthode de culture et traitement des explants

A réception, les explants ont été dégraissés. Des disques de peau circulaires d'un diamètre de 1 cm ont été obtenus à l'aide d'un emporte-pièce. Dans une plaque six puits, une grille en acier inoxydable de 1,6 cm de diamètre est placée dans 5 ml de milieu de culture. Chaque explant en culture est déposé sur sa grille de manière à baigner dans le milieu de culture tout en laissant l'épiderme à l'air. Les explants ont été cultivés pendant 48 heures sans changement du milieu de culture. Les différents traitements ont été réalisés par un dépôt topique de 10 µL des préparations à tester à J0 et à J1. Chaque condition expérimentale a été répétée sur 3 punchs.

Des explants contrôles ont été cultivés de la même manière que les explants traités. Les traitements ont été réalisés par un dépôt topique de 10 µL du solvant TRIGLYCERIDES à la concentration de 0,05%.

### - Immunomarquages in situ

A la fin du temps de culture, les explants ont été recueillis puis divisés en deux parties égales à l'aide d'un scalpel. Une moitié a été congelée dans du tissu Teck^{™} (Sakura) à l'aide d'isopentane refroidi dans de l'azote liquide puis conservée à -80° C en attente de l'immunomarquage. La seconde moitié a été conservée dans du formol tamponné à 4%.

Des coupes transversales de 5 µm d'épaisseur de chaque explant ont été réalisées à l'aide d'un cryotome. Les coupes ont été séchées à l'air ambiant. Après réhydratation dans du PBS, les coupes ont été incubées pendant 1 heure avec la solution d'anticorps primaire préparée dans une solution (PBS- BSA-Tween). Après lavages successifs, les anticorps primaires ont été révélés par un anticorps secondaire couplé à une sonde FITC au 1/100ème de la forme commerciale. Les noyaux cellulaires ont été marqués par le colorant fluorescent DAPI.

Des lames poly-lysine ont été utilisées pour recueillir les sections de peau.

### - Observations microscopiques

Chaque section de peau a été observée à l'aide d'un microscope à épifluorescence Olympus BX60 au grossissement 20X. Pour chaque section, deux photographies ont été réalisées à l'aide d'une caméra Olympus DP72^{®} pilotée par le logiciel Cell F. Les clichés ont été identifiés de la manière suivante : Date du marquage - Condition de traitement - Numéro de l'image - Marqueur. La contre coloration des noyaux a été réalisée à l'aide du marqueur nucléaire DAPI. Par un glissement de teinte à l'aide d'un logiciel de traitement d'image, la couleur des noyaux a été transformée du bleu au rouge. Pour chaque condition de traitement des épidermes, un scoring visuel portant sur l'intensité du marquage a été réalisé au grossissement x10. L'échelle d'évaluation était la suivante :

| | |
|---|---|
| Marquage très faible | **1** |
| Marquage faible | **2** |
| Marquage modéré | **3** |
| Marquage fort | **4** |

Les données obtenues ont été analysées, présentées sous forme d'histogrammes en comparaison aux conditions contrôle solvant Triglycérides.

### -Résultats

L'ensemble des marquages était localisé au niveau des couches supérieures de l'épiderme (spineuse et/ou granuleuse et/ou cornée de l'épiderme).

### - Immunomarquage Filaggrine

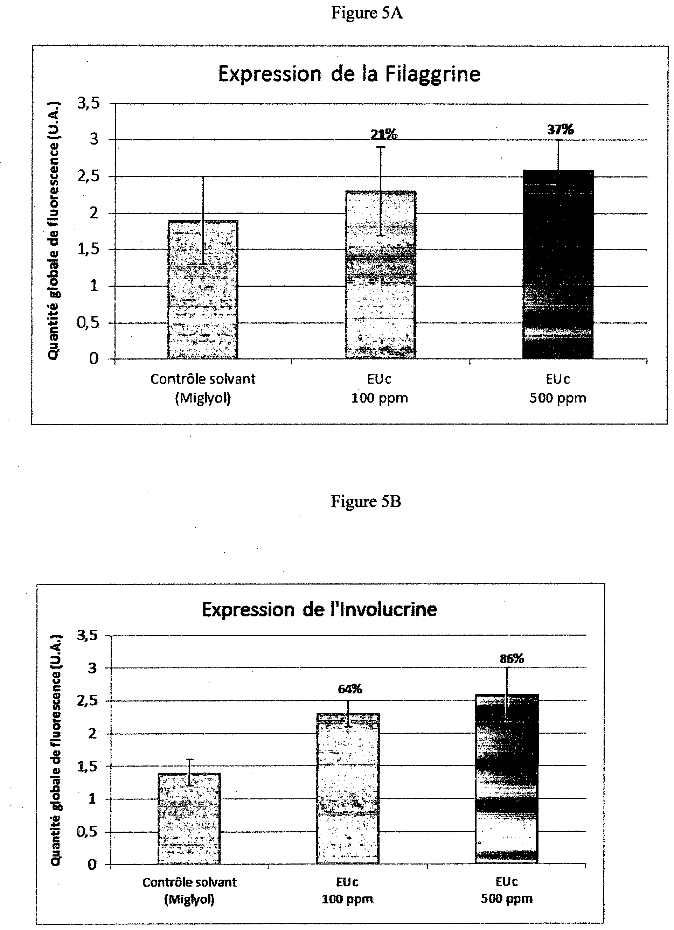

Les résultats des scorings réalisés sont présentés dans le graphique de la figure 5A.

L'analyse des données d'expression du marquage Filaggrine indique une augmentation dose dépendante de la quantité de fluorescence pour les explants traités avec l'extrait d'Uchuva à 0,01% (+21%) et 0,05% (+37%) en comparaison au groupe Contrôle Solvant.

### - Immunomarquage Involucrine

Les résultats des scorings réalisés sont présentés dans le graphique de la figure 5B. L'analyse des données d'expression du marquage INVOLUCRINE indique une augmentation significative dose dépendante de la quantité de fluorescence pour les explants traités avec l'extrait d'Uchuva à 0,01% (+64%) et 0,05% (+86%) en comparaison au groupe Contrôle.

### - Conclusion

Dans cette étude, les résultats ont montré que le traitement topique d'explants de peau en culture pendant 48h avec l'extrait d'Uchuva a induit une augmentation de l'expression de protéines impliquées dans l'hydratation et l'effet barrière de la peau (respectivement la filaggrine et l'involucrine). L'augmentation d'expression induite par l'extrait d'Uchuva a été plus importante pour l'involucrine que pour la filaggrine. Le maximum d'expression de ces protéines a toujours été observé à la concentration de 0,05%. D'après ces résultats, l'extrait d'Uchuva selon l'invention présente un profil d'activité hydratante et structurante de l'épiderme.

### Exemple 23 : Tests démontrant le pouvoir dépigmentant de la peau par l'extrait d'Uchuva

Le potentiel effet dépigmentant de l'extrait d'Uchuva a été recherché dans un modèle de mélanocytes épidermiques humains normaux (NHEM). L'effet de l'extrait d'Uchuva sur la synthèse de mélanine a été évalué après 10 jours d'incubation en condition stimulée par la L-tyrosine. Dans ce test, la molécule inhibitrice de référence est l'acide lipoïque, dont le mécanisme d'action passe par une inhibition de l'expression du facteur de transcription MITF (microphthalmia-associated transcription factor). Ce facteur de transcription est le régulateur majeur de l'expression de la tyrosinase et son inhibition a pour conséquence de diminuer l'expression de la tyrosinase, enzyme impliquée dans la synthèse de mélanine. Les mélanocytes ont été ensemencés en plaque 24 puits et cultivés en milieu de culture pendant 24 heures. Le milieu a ensuite été remplacé par du milieu de culture (Medium 254 complémenté avec PMA free HMGS-2 + Pénicilline 50 U/ml - Streptomycine 50 µg/ml) supplémenté en L-tyrosine (à 1 mM) et contenant ou non (témoin stimulé) le composé ou la référence (acide lipoïque testée à 5 µg/ml). Une condition témoin non stimulé a été réalisée en parallèle. Les cellules ont ensuite été incubées pendant une durée totale de 10 jours, avec renouvellements des traitements après 3 et 7 jours d'incubation. Après incubation, la mélanine a été extraite par lyse des cellules avec une solution de NaOH 0.5 N. La densité optique (DO) des échantillons a été mesurée à 405 nm, puis la quantité de mélanine a été déterminée en comparaison avec une gamme de mélanine exogène (courbe de mélanine incluant des standards de 0.39 à 100 µg/ml). Les résultats ont été exprimés en µg/ml de mélanine, en pourcentage de témoin stimulé et en pourcentage d'inhibition. Le traitement des mélanocytes épidermiques humains normaux (NHEM) par la L-tyrosine, testée à 1 mM, a fortement stimulé la synthèse de mélanine. La référence, acide lipoïque, testé à 5 µg/ml, a fortement inhibé la stimulation induite par la L-tyrosine (108% d'inhibition). Cet effet était attendu et a permis de valider le test.

Les concentrations à tester de l'extrait d'Uchuva dans l'étude ont été sélectionnées suite à un test préliminaire de cytotoxicité :

| | |
|---|---|
| - Type cellulaire : | NHEM en milieu de culture |
| - Temps d'incubation : | 72 + 96 + 72 heures |
| - Paramètres d'évaluation : | Réduction du MTT et observations morphologiques au microscope. |
| | A la fin du traitement, les cellules ont été incubées en présence de MTT (sel de tétrazolium) dont la transformation en cristaux bleus de formazan est proportionnelle à l'activité de la succinate deshydrogénase (enzyme mitochondriale). Après dissociation des cellules et solubilisation du formazan par ajout de DMSO, la |

| | |
|---|---|
| | densité optique (DO), représentative du nombre de cellules vivantes et de leur activité métabolique, a été mesurée avec un lecteur de microplaques à 540 nm (VERSAmax, Molecular Devices). |

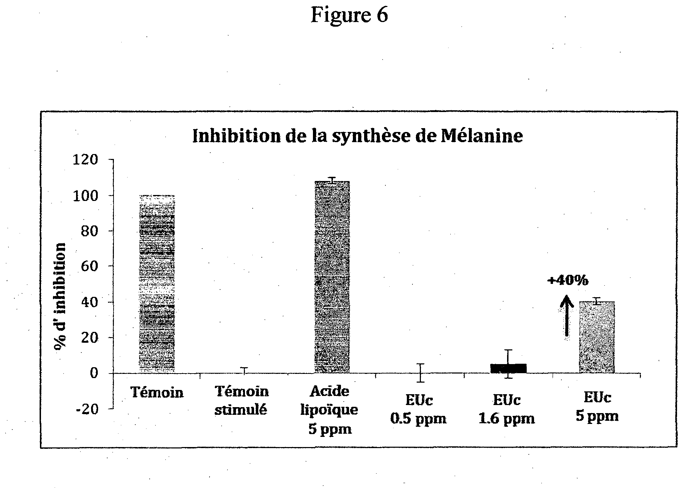

Les concentrations ainsi retenues pour la suite de l'étude sont : 0.5 - 1.6 - 5 µg/ml correspondant respectivement à 5.10⁻⁵, 16.10⁻⁵ et 5.10⁻⁵%. Dans les conditions expérimentales de cette étude, l'extrait d'Uchuva, testé à 5 µg/ml, a modérément inhibé la synthèse de mélanine (40% d'inhibition). Testé à des concentrations plus faibles, 0.5 et 1.6 µg/ml, le composé n'a pas présenté d'effet. Les résultats sont présentés dans le diagramme de la figure 6. Dans les conditions expérimentales de cette étude, l'extrait d'Uchuva a présenté un effet dépigmentant modéré et qui a été observé seulement à la plus forte concentration testée (5 µg/ml).

Le tableau ci-dessous présente la correspondance des concentrations de l'extrait d'Uchuva testé dans les exemples 21 à 23 (ppm et % en poids par rapport à la concentration totale de la composition.

| **CIBLES** | **CONC. TESTEES ppm** | **CONC. TESTEES %** |
|---|---|---|
| Collagène I | 0,8 | 8.10-5 |
| | 1,6 | 16.10-5 |
| | 3,2 | 32.10-5 |
| Collagène I | 0,5 | 5.10-5 |
| | 1 | 10.10-5 |
| | 2 | 20.10-5 |
| Collagène III | 0,8 | 8.10-5 |
| | 1,6 | 16.10-5 |
| Collagène IV | 3,2 | 32.10-5 |
| | 0,4 | 4.10-5 |
| | 0,8 | 8.10-5 |
| Elastine | 0,5 | 5.10-5 |
| | 1 | 10.10-5 |
| | 2 | 20.10-5 |
| Fibrilline-1 | 0,5 | 5.10-5 |
| | 1 | 10.10-5 |
| Niveau de Méthylation | 2 | 20.10-5 |
| | 0,5 | 5.10-5 |
| | 1 | 10.10-5 |
| | 2 | 20.10-5 |
| Filaggrine | 100 | 0,01 |
| Involucrine | 500 | 0,05 |
| | 100 | 0,01 |
| | 500 | 0,05 |
| Mélanine | 0,5 | 5.10-5 |
| | 1,6 | 16.10-5 |
| | 5 | 50.10-5 |

### Références bibliographiques

1. Les esters de sucres : voies de synthèse et potentialités d'utilisation, Piccicuto et al. Biotechnol. Agron. Soc. Environ. 2001, 5, 209-219
2. Domaines d'applications des sucroesters et sucroglycerides, Mireille Cecchin, DESS ingénierie documentaire, rapport bibliographique, 2001.
3. Preparative isolation and structural characterization of sucrose ester isomers from oriental tobacco, Jia et al. Carbohydrate Research 2013, 372, 73-77
4. Characterization of 2,3,4,3'-tetra-O-acylated sucrose esters associated with the glandular trichomes of Lycopersicon typicum King et al. J. Agric. Food Chem. 1993, 41, 469-473
5. New Multidrug résistance modulators from Atractylodis lanceae RhizomaMurakami et al. Bioorg. Med. Chem. Lett. 2000, 10, 2629-2632
6. Labdanes ans sucrose Ester from Physalis sórdida, Maldonado et al. J. Nat. Prod. 2006, 69, 1511-1513
7. Oligosaccharide esters from the roots of Polygala arillata, Kobayashi et al. J. Nat. Prod. 2000, 63, 1066-1069
8. Synthesis and antitumor activity of lapathoside D and its analogs, Panda et al. Eur. J. Med. Chem. 2012, 53, 1-12
9. Direct inhibition of elastase and matrix metalloproteinases and stimulation of biosynthesis of fibrillar collagens, elastin, and fibrillins by xanthohumol (Journal of Cosmetic Science, Volume 61, Issue 2, Pages125-132, Journal 2010.)
10. From elastin to elastic fibers, part I. The *in vitro* effects of a naturel dipeptide on the biological cascade.
11. A novel anti-ageing mechanism for retinol: induction of dermal elastin synthesis and elastin fibre formation International Journal of Cosmetic Science Volume 33 Issue 1 Pages 62-69 Journal 2011
12. Matrix proteins of the papillary dermis - primary targets of intrinsic dermal aging? Global Ingrédients & Formulations Guide 2009 Pages131-142 Conférence 2009
13. Matrix proteins of the papillary dermis - primary targets of intrinsic dermal aging? IFSCC Magazine Volume 11 Issue 3 Pages 225-229 Journal 2008
14. Fibrillines et fibrillinopathies, Gwenaëlle Collod et al., Médecine et Science n°10, vol. 12, p. 1077 -1086, octobre 1996
15. Les filaments perlés : structure, fonctions et maladies associées, Eric Hanssen et al., Médecine et Science n°3, vol. 17, p. 327 -335, mars 2001
16. Stiff skin syndrome cause found, P.J. Couke et al., 18 mars 2010
17. Fibrillin Assembly Requires Fibronectin, L. Sabatier et al., Molecular Biology of the Cell, Vol. 20, 846-858, February 1, 2009.
18. Dissecting the Fibrillin Microfibril: Structural Insights into Organization and Function, S. A. Jensen et al., Structure Review 20, Cell Press, February 8, 2012
19. Type VII collagen gene expression by human skin fibroblasts and keratinocytes in culture: influence of donor age and cytokine responses, Y.K. Chen et al., J Invest Dermatol. 1994 Feb;102(2):205-9.
20. WO 2001/007006 A1, ASSOCIATION OF FIBRILLIN AND A CYANOPHYTA EXTRACT, PREPARATION METHOD AND USE AS MEDICINE, PIERRE FABRE DERMO-COSMETIQUE
21. Epidermal growth factor and multiplication of cultured human epidermal keratinocytes, Nature 1977, 265, 421-423
22. Retinoid-Responsive transcriptional changes in epidermal Keratinocytes, Cellular Physiology 2009, 220, 427-439
23. Silybin from Silybum Marianum Seeds inhibits confluent-Induced keratinocytes différentiation as effectively as retinoic acid without inducing inflammatory cytokine, Journal of clinical biochemistry and nutrition, 2009, 45, 178-184.
24. Epigenetic-aging-signature to détermine age in different tissues, Carmen M. Koch and Wolfgang Wagner, AGING october 2011, Vol. 3 No 10)
25. Aging is associated with highly defined epigenetic changes in the human epidermis, Raddatz et al. Epigenetics & Chromatin 2013, 6:36
26.Aging, rejuvenation and epigenetic reprogramming : resetting the aging clock, Rando TA, Chang HY, the Glenn Laboratories for the Biology of Aging, Stanford University School of Medicine, Cell. 2012 Jan 2; 148(1-2):46-57.doi: 10.1016/j.cell.2012.01.003.
27. Epigenetic control of aging, Muñoz-Najar U., Sedivy JM., Department of Molecula Biology, Cell Biology and Biochemistry, Brown University, Providence, Rhode Island USA, Antioxid Redox Signal 2011 Jan 15; 14(2):241-59. Doi: 10.1089/ars.2010.3250.Epub 2010 Nov 22.
28. Epigenetics and aging, D'Aquila P., Rose G., Bellizzi D., Passarino G., Department of Cell Biology, University of Calabria, Rende Italy, Maturitas 2013 Feb; 74(2): 130-6. Doi: 10.1016/j.maturitas.2012.11.005. Epub 2012 Dec 12.
29. Epigenetic factors in aging and longevity, Gravina S., Vijq. J., Depratment of Genetics, Albert Einstein College of Medicine, Bronx New-York USA, Pflugers Arch. 2010 Jan; 459(2): 247-58. Doi: 10.1007/s00424-009-0730-7. Epub 2009 Sep 19.
30. Modulation of gene expression as a new skin anti-aging strategy, Talbourdet S., Sadick NS., Lazou K. et co., LVMH Recherche - Parfums Christian Dior, France, J. Drugs Dermatol. 2007 Jun;6(6 suppl):s25-33.
31. Experimental approaches to the study of epigenomic dysregulation in ageing, Thompson RF., Fazzari MJ., Greally JM., Department of Genetics and Center for Epigenomics, Albert Einstein College of Medicine, Bronx New-York USA, Exp.Gerontol. 2010 Apr;45(4):225-68. Doi: 10.1016/j.exger.2009.12.013. Epub 2010 Jan 10.
32. KH Choi et al, Exp Mol Med, 37, 546, 2005.
33. Ginger RS et al., Arch Dermatol Res 297 :235-241, 2005; Rawlings AV and Matts PJ, JID, 124 :1099-1110, 2005.
34. Revue dans Proksch E et al, Exp Dermatol, 17 :1063-1072, 2008.
35. Hitomi K. Transglutaminases in skin epidermis, Eur J Dermatol, 2005,15 :313.

## Revendications

1. Sucrose-esters comprenant principalement un ou plusieurs sucrose-esters moyennement polaires à apolaires ayant un nombre de carbones des groupements acyles de C1 à C10 **caractérisés en ce qu'**ils sont obtenus d'un extrait végétal provenant du calice de *Physalis peruviana* ou sont synthétisés, les sucrose-esters étant choisis parmi:
- le 3-O-(2-methyl-1-oxopropyl)-β-D-fructofuranosyle(1→2)-3,4-bis(2-methylpropanoate)-2-decanoate-α-D-glucopyranoside,
- le 3-O-(3-methyl-1-oxobutyl)-β-D-fructofuranosyle(1→2)-3,4-bis(2-methylpropanoate)-2-decanoate-α-D-glucopyranoside,
- le 3-O-(3-methyl-1-oxobutyl)-β-D-fructofuranosyle(1→2)-3-(2-methylpropanoate)-2-decanoate-α-D-glucopyranoside,
- le 3-O-(2-methyl-1-oxopropyl)-β-D-fructofuranosyle(1→2)-3,6-bis(2-methylpropanoate)-2-decanoate-α-D-glucopyranoside,
- le 3-O-(2-methyl-1-oxopropyl)-β-D-fructofuranosyle(1→2)-3-(3-methylbutanoate)-2-decanoate-α-D-glucopyranoside.
- le 3-O-(2-methyl-1-oxopropyl)-β-D-fructofuranosyle(1→2)-3,4-bis(2-methylpropanoate)-2-nonanoate-α-D-glucopyranoside,
- le 3-O-(2-methyl-1-oxopropyl)-β-D-fructofuranosyle(1→2)-3,4-bis(2-methylpropanoate)-2-octanoate-α-D-glucopyranoside,
pour leur utilisation dans le traitement dermatologique des pathologies impliquant une sécheresse pathologique de la peau, des muqueuses ou des phanères, pour dépigmenter la peau et pour favoriser la cicatrisation.

2. Composition cosmétique **caractérisée en ce qu'**elle comprend comme principe actif, dans un milieu physiologique adapté, les sucrose-esters comprenant principalement un ou plusieurs sucrose-esters moyennement polaires à apolaires ayant un nombre de carbones des groupements acyles de C1 à C10 obtenus d'un extrait végétal provenant du calice de *Physalis peruviana* ou synthétisés, les sucrose-esters étant choisis parmi:
- le 3-O-(2-methyl-1-oxopropyl)-β-D-fructofuranosyle(1→2)-3,4-bis(2-methylpropanoate)-2-decanoate-α-D-glucopyranoside,
- le 3-O-(3-methyl-1-oxobutyl)-β-D-fructofuranosyle(1→2)-3,4-bis(2-methylpropanoate)-2-decanoate-α-D-glucopyranoside,
- le 3-O-(3-methyl-1-oxobutyl)-β-D-fructofuranosyle(1→2)-3-(2-methylpropanoate)-2-decanoate-α-D-glucopyranoside,
- le 3-O-(2-methyl-1-oxopropyl)-β-D-fructofuranosyle(1→2)-3,6-bis(2-methylpropanoate)-2-decanoate-α-D-glucopyranoside,
- le 3-O-(2-methyl-1-oxopropyl)-β-D-fructofuranosyle(1→2)-3-(3-methylbutanoate)-2-decanoate-α-D-glucopyranoside.
- le 3-O-(2-methyl-1-oxopropyl)-β-D-fructofuranosyle(1→2)-3,4-bis(2-methylpropanoate)-2-nonanoate-α-D-glucopyranoside,
- le 3-O-(2-methyl-1-oxopropyl)-β-D-fructofuranosyle(1→2)-3,4-bis(2-methylpropanoate)-2-octanoate-α-D-glucopyranoside.

3. Composition cosmétique selon la revendication 2 **caractérisée en ce que** le principe actif est présent à une concentration comprise entre 10⁻⁶ et 50 % en poids par rapport au poids total de la composition.

4. Composition cosmétique selon la revendication 2 ou 3 **caractérisée en ce qu'**elle est adaptée à une administration topique et se présente notamment sous forme d'une solution aqueuse, hydro-alcoolique ou huileuse ou sous la forme d'une émulsion huile-dans-eau, eau-dans-huile ou émulsions multiples ou sous forme de crèmes, de suspensions, ou encore de poudres ; ladite composition pouvant être plus ou moins fluide ou solide et avoir l'aspect d'une crème, d'une lotion, d'un lait, d'un shampooing, d'un sérum, d'une pommade, d'un gel, d'une pâte, d'une mousse ou d'un stick.

5. Procédé de traitement non thérapeutique pour améliorer l'aspect de la peau, des muqueuses ou des phanères, pour prévenir et/ou lutter contre la sécheresse de la peau et des muqueuses, pour prévenir et/ou à lutter contre les signes cutanés du vieillissement et/ou du photo-vieillissement, pour lutter contre la perte d'élasticité et de fermeté de la peau et pour dépigmenter la peau, consistant à appliquer sur la surface de la peau et/ou des cheveux, une quantité efficace de la composition cosmétique selon l'une quelconque des revendications 2 à 4.

6. Composition nutracosmétique à ingérer comprenant une quantité efficace comprise entre 10⁻⁶ et 50 % en poids par rapport au poids total de la composition de sucrose-esters comprenant principalement un ou plusieurs sucrose-esters moyennement polaires à apolaires ayant un nombre de carbones des groupements acyles de C1 à C10 obtenus d'un extrait végétal provenant du calice de *Physalis peruviana* ou synthétisés, les sucrose-esters étant choisis parmi:
- le 3-O-(2-methyl-1-oxopropyl)-β-D-fructofuranosyle(1→2)-3,4-bis(2-methylpropanoate)-2-decanoate-α-D-glucopyranoside,
- le 3-O-(3-methyl-1-oxobutyl)-β-D-fructofuranosyle(1→2)-3,4-bis(2-methylpropanoate)-2-decanoate-α-D-glucopyranoside,
- le 3-O-(3-methyl-1-oxobutyl)-β-D-fructofuranosyle(1→2)-3-(2-methylpropanoate)-2-decanoate-α-D-glucopyranoside,
- le 3-O-(2-methyl-1-oxopropyl)-β-D-fructofuranosyle(1→2)-3,6-bis(2-methylpropanoate)-2-decanoate-α-D-glucopyranoside,
- le 3-O-(2-methyl-1-oxopropyl)-β-D-fructofuranosyle(1→2)-3-(3-methylbutanoate)-2-decanoate-α-D-glucopyranoside.
- le 3-O-(2-methyl-1-oxopropyl)-β-D-fructofuranosyle(1→2)-3,4-bis(2-methylpropanoate)-2-nonanoate-α-D-glucopyranoside,
- le 3-O-(2-methyl-1-oxopropyl)-β-D-fructofuranosyle(1→2)-3,4-bis(2-methylpropanoate)-2-octanoate-α-D-glucopyranoside, seuls ou en association avec d'autres principes actifs, destinée à améliorer l'aspect de la peau, des muqueuses ou des phanères, à prévenir et/ou lutter contre la sécheresse de la peau et des muqueuses, à prévenir et/ou à lutter contre les signes cutanés du vieillissement et/ou du photo-vieillissement.

7. Procédé de préparation d'un mélange de sucrose-esters provenant d'un extrait végétal selon la revendication 1, 2 ou 6, comprenant les étapes suivantes:
- séchage de la partie de plante,
- broyage,
- extraction avec le dioxyde de carbone supercritique sans ou avec co-solvant tel qu'un alcool ou une huile végétale ou encore un ester,
- éventuellement une désolvantation, puis
- éventuellement une purification avec une ou plusieurs méthodes appropriées.

## Patentansprüche

1. Saccharoseester, die hauptsächlich einen oder mehrere mittelpolare bis unpolare Saccharoseester umfassen, wobei die Anzahl an Kohlenstoffen von deren azyklischen Gruppen C1 bis C10 beträgt, **dadurch gekennzeichnet, dass** sie aus einem Pflanzenextrakt gewonnen werden, der aus dem Kelch von *Physalis peruviana* stammt, oder synthetisiert werden, wobei die Saccharoseester aus den folgenden ausgewählt sind:
- 3-O-(2-Methyl-1-oxopropyl)-β-D-fructofuranosyl(1→2)-3,4-bis(2-methylpropanoat)-2-decanoat-α-D-glucopyranosid,
- 3-O-(3-Methyl-1-oxobutyl)-β-D-fructofuranosyl(1→2)-3,4-bis(2-methylpropanoat)-2-decanoat-α-D-glucopyranosid,
- 3-O-(3-Methyl-1-oxobutyl)-β-D-fructofuranosyl(1→2)-3-(2-methylpropanoat)-2-decanoat-α-D-glucopyranosid,
- 3-O-(2-Methyl-1-oxopropyl)-β-D-fructofuranosyl(1→2)-3,6-bis(2-methylpropanoat)-2-decanoat-α-D-glucopyranosid,
- 3-O-(2-Methyl-1-oxopropyl)-β-D-fructofuranosyl(1→2)-3-(3-methylbutanoat)-2-decanoat-α-D-glucopyranosid.
- 3-O-(2-Methyl-1-oxopropyl)-β-D-fructofuranosyl(1→2)-3,4-bis(2-methylpropanoat)-2-nonanoat-α-D-glucopyranosid,
- 3-O-(2-Methyl-1-oxopropyl)-β-D-fructofuranosyl(1→2)-3,4-bis(2-methylpropanoat)-2-octanoat-α-D-glucopyranosid,
um diese in der dermatologischen Behandlung von Erkrankungen zu verwenden, die mit einer krankhaften Trockenheit der Haut, der Schleimhäute oder der Hautanhangsgebilde einhergehen, um die Haut zu depigmentieren und um die Wundheilung zu begünstigen.

2. Kosmetische Zusammensetzung, **dadurch gekennzeichnet, dass** sie als Wirkstoff, in einem geeigneten physiologischen Medium, diejenigen Saccharoseester umfasst, welche hauptsächlich einen oder mehrere mittelpolare bis unpolare Saccharoseester umfassen, wobei die Anzahl an Kohlenstoffen von deren azyklischen Gruppen C1 bis C10 beträgt und sie aus einem Pflanzenextrakt gewonnen werden, der aus dem Kelch von *Physalis peruviana* stammt, oder synthetisiert werden, wobei die Saccharoseester aus den folgenden ausgewählt sind:
- 3-O-(2-Methyl-1-oxopropyl)-β-D-fructofuranosyl(1→2)-3,4-bis(2-methylpropanoat)-2-decanoat-α-D-glucopyranosid,
- 3-O-(3-Methyl-1-oxobutyl)-β-D-fructofuranosyl(1→2)-3,4-bis(2-methylpropanoat)-2-decanoat-α-D-glucopyranosid,
- 3-O-(3-Methyl-1-oxobutyl)-β-D-fructofuranosyl(1→2)-3-(2-methylpropanoat)-2-decanoat-α-D-glucopyranosid,
- 3-O-(2-Methyl-1-oxopropyl)-β-D-fructofuranosyl(1→2)-3,6-bis(2-methylpropanoat)-2-decanoat-α-D-glucopyranosid,
- 3-O-(2-Methyl-l-oxopropyl)-β-D-fructofuranosyl(1→2)-3-(3-methylbutanoat)-2-decanoat-α-D-glucopyranosid,
- 3-o-(2-Methyl-1-oxopropyl)-β-D-fructofuranosyl(1→2)-3,4-bis(2-methylpropanoat)-2-nonanoat-α-D-glucopyranosid,
- 3-O-(2-Methyl-l-oxopropyl)-β-D-fructofuranosyl(1→2)-3,4-bis(2-methylpropanoat)-2-octanoat-α-D-glucopyranosid.

3. Kosmetische Zusammensetzung nach Anspruch 2, **dadurch gekennzeichnet, dass** der Wirkstoff in einer Konzentration vorliegt, die im Bereich von 10⁻⁶ bis 50 Gewichts-% liegt, bezogen auf das Gesamtgewicht der Zusammensetzung.

4. Kosmetische Zusammensetzung nach Anspruch 2 oder 3, **dadurch gekennzeichnet, dass** sie sich für eine topische Verabreichung eignet und insbesondere in Form einer wässrigen, wässrig-alkoholischen oder ölbasierten Lösung oder in Form einer Emulsion des Typs Öl-in-Wasser, Wasser-in-Öl oder von Mehrfachemulsionen oder in Form von Cremes, Suspensionen oder auch Pulvern vorliegt; wobei die Zusammensetzung mehr oder weniger fließfähig oder feststofflich sein kann und das Erscheinungsbild einer Creme, einer Lotion, einer Milch, eines Shampoos, eines Serums, einer Salbe, eines Gels, einer Paste, eines Schaums oder eines Sticks haben kann.

5. Verfahren zur nicht-therapeutischen Behandlung, um das Aussehen der Haut, der Schleimhäute oder der Hautanhangsgebilde zu verbessern, einer Trockenheit der Haut und der Schleimhäute vorzubeugen und/oder diese zu bekämpfen, um den hautbezogenen Anzeichen der Alterung und/oder der lichtbedingten Alterung vorzubeugen und/oder diese zu bekämpfen, um den Verlust an Elastizität und Straffheit der Haut zu bekämpfen und um die Haut zu depigmentieren, wobei es darin besteht, eine wirksame Menge der kosmetischen Zusammensetzung nach einem beliebigen der Ansprüche 2 bis 4 auf die Oberfläche und/oder die Haare aufzubringen.

6. Nutrakosmetische Zusammensetzung zur Einnahme, die eine wirksame Menge, welche im Bereich von 10⁻⁶ bis 50 Gewichts-% unter Bezugnahme auf das Gesamtgewicht der Zusammensetzung liegt, an Saccharoseestern umfasst, welche hauptsächlich einen oder mehrere mittelpolare bis unpolare Saccharoseester umfassen, wobei die Anzahl an Kohlenstoffen von deren azyklischen Gruppen C1 bis C10 beträgt und sie aus einem Pflanzenextrakt gewonnen werden, der aus dem Kelch von *Physalis peruviana* stammt, oder synthetisiert werden, wobei die Saccharoseester aus den folgenden ausgewählt sind:
- 3-O-(2-Methyl-l-oxopropyl)-β-D-fructofuranosyl(1→2)-3,4-bis(2-methylpropanoat)-2-decanoat-α-D-glucopyranosid,
- 3-O-(3-Methyl-1-oxobutyl)-β-D-fructofuranosyl(1→2)-3,4-bis(2-methylpropanoat)-2-decanoat-α-D-glucopyranosid,
- 3-O-(3-Methyl-1-oxobutyl)-β-D-fructofuranosyl(1→2)-3-(2-methylpropanoat)-2-decanoat-α-D-glucopyranosid,
- 3-O-(2-Methyl-1-oxopropyl)-β-D-fructofuranosyl(1→2)-3,6-bis(2-methylpropanoat)-2-decanoat-α-D-glucopyranosid,
- 3-O-(2-Methyl-1-oxopropyl)-β-D-fructofuranosyl(1→2)-3-(3-methylbutanoat)-2-decanoat-α-D-glucopyranosid,
- 3-O-(2-Methyl-1-oxopropyl)-β-D-fructofuranosyl(1→2)-3,4-bis(2-methylpropanoat)-2-nonanoat-α-D-glucopyranosid,
- 3-O-(2-Methyl-1-oxopropyl)-β-D-fructofuranosyl(1→2)-3,4-bis(2-methylpropanoat)-2-octanoat-α-D-glucopyranosid, allein oder in Verbindung mit weiteren Wirkstoffen, wobei sie dazu bestimmt ist, das Aussehen der Haut, der Schleimhäute oder der Hautanhangsgebilde zu verbessern, einer Trockenheit der Haut und der Schleimhäute vorzubeugen und/oder diese zu bekämpfen, den hautbezogenen Anzeichen der Alterung und/oder der lichtbedingten Alterung vorzubeugen und/oder diese zu bekämpfen.

7. Verfahren zur Herstellung einer Mischung aus Saccharoseestern, die aus einem Pflanzenextrakt stammt, nach Anspruch 1, 2 oder 6, wobei es die folgenden Schritte umfasst:
- Trocknen des Pflanzenteils,
- Zerkleinern,
- Extrahieren mit überkritischem Kohlendioxid ohne oder mit einem Hilfslösungsmittel wie etwa einem Alkohol und einem Pflanzenöl oder auch einem Ester,
- möglicherweise Entfernen der Lösungsmittel, und danach
- möglicherweise Aufreinigen mittels einer oder mehrerer geeigneter Methoden.

## Claims

1. Sucrose esters mainly comprising one or more moderately polar to non-polar sucrose esters having a carbon number of acyl groups from C1 to C10, **characterized in that** they are obtained from a plant extract from the calyx of *Physalis peruviana* or are synthetised, the sucrose esters being selected from:
the 3-O-(2-methyl-1-oxopropyl)β-D-fructofuranosyl(1→2)-3,4-bis(2-methylpropanoate)-2-decanoate-α-D-glucopyranoside,
the 3-O-(3-methyl-1-oxobutyl)-β-D-fructofuranosyl(1→2)-3,4-bis(2-methylpropanoate)-2-decanoate-α-D-glucopyranoside,
the 3-O-(3-methyl-1-oxobutyl)-β-D-fructofuranosyl(1→2)-3-(2-methylpropanoate)-2-decanoate-α-D-glucopyranoside,
the 3-O-(2-methyl-1-oxopropyl)-β-D-fructofuranosyl(1→2)-3,6-bis(2-methylpropanoate)-2-decanoate-α-D-glucopyranoside,
the 3-O-(2-methyl-1-oxopropyl)-β-D-fructofuranosyl(1→2)-3-(3-methylbutanoate)-2-decanoate-α-D-glucopyranoside.
the 3-O-(2-methyl-1-oxopropyl)-β-D-fructofuranosyl(1→2)-3,4-bis(2-methylpropanoate)-2-nonanoate -α-D-glucopyranoside,
the 3-O-(2-methyl-1-oxopropyl)-β-D-fructofuranosyl(1→2)-3,4-bis(2-methylpropanoate)-2-octanoate-α-D-glucopyranoside
for use in the dermatological treatment of diseases involving pathological dryness of skin, skin appendages or mucous membranes, for depigmenting skin and for promoting would healing.

2. Cosmetic composition, **characterized in that** it comprises, as active ingredient, in a suitable physiological medium, the sucrose esters mainly comprising one or more moderately polar to non-polar sucrose esters having a carbon number of acyl groups from C1 to C10, obtained from a plant extract from the calyx of *Physalis peruviana* or are synthetised, the sucrose esters being selected from:
the 3-O-(2-methyl-1-oxopropyl)-β-D-fructofuranosyl(1→2)-3,4-bis(2-methylpropanoate)-2-decanoate-α-D-glucopyranoside,
the 3-O-(3-methyl-1-oxobutyl)-β-D-fructofuranosyl(1→2)-3,4-bis(2-methylpropanoate)-2-decanoate-α-D-glucopyranoside,
the 3-O-(3-methyl-1-oxobutyl)-β-D-fructofuranosyl(1→2)-3-(2-methylpropanoate)-2-decanoate-α-D-glucopyranoside,
the 3-O-(2-methyl-1-oxopropyl)-β-D-fructofuranosyl(1→2)-3,6-bis(2-methylpropanoate)-2-decanoate-α-D-glucopyranoside,
the 3-O-(2-methyl-1-oxopropyl)-β-D-fructofuranosyl(1→2)-3-(3-methylbutanoate)-2-decanoate-α-D-glucopyranoside.
the 3-O-(2-methyl-1-oxopropyl)-β-D-fructofuranosyl(1→2)-3,4-bis(2-methylpropanoate)-2-nonanoate -α-D-glucopyranoside,
the 3-O-(2-methyl-1-oxopropyl)-β-D-fructofuranosyl(1→2)-3,4-bis(2-methylpropanoate)-2-octanoate-α-D-glucopyranoside.

3. Composition according to claim 2, **characterized in that** the active ingredient is present at a concentration between 10⁻⁶ and 50% by weight with respect to the total weight of the composition.

4. Cosmetic composition according to claim 2 or 3, **characterized in that** it is suitable for a topical administration and exists, inter alia, in the form of an aqueous, hydroalcoholic or oily solution or in the form of an oil-in-water or water-in-oil emulsion or multiple emulsions or in the form of creams, suspensions, or even powders; the said composition can be more or less fluid or solid and look like a cream, lotion, milk, shampoo, serum, ointment, gel, paste, foam or a stick.

5. Non-therapeutic treatment process to improve the appearance of the skin, mucous membranes or skin appendages, prevent and/or fight against the dryness of skin and mucous membranes, prevent and/or fight against the cutaneous signs of ageing and/or photo ageing, fight against the loss of elasticity and firmness of skin and depigment skin, the process comprising applying on the surface of the skin and/or hair an effective quantity of the cosmetic composition according to any one of claims 2 to 4.

6. Nutracosmetic composition to be ingested, comprising an effective amount comprised between 10⁻⁶ and 50% by weight with respect to the total weight of the composition of sucrose esters mainly comprising one or more moderately polar to non-polar sucrose esters having a carbon number of acyl groups from C1 to C10, which are obtained from a plant extract from the calyx of *Physalis peruviana* or synthetised, the sucrose esters being selected from:
the 3-O-(2-methyl-1-oxopropyl)-β-D-fructofuranosyl(1→2)-3,4-bis(2-methylpropanoate)-2-decanoate-α-D-glucopyranoside,
the 3-O-(3-methyl-1-oxobutyl)-β-D-fructofuranosyl(1→2)-3,4-bis(2-methylpropanoate)-2-decanoate-α-D-glucopyranoside,
the 3-O-(3-methyl-1-oxobutyl)-β-D-fructofuranosyl(1→2)-3-(2-methylpropanoate)-2-decanoate-α-D-glucopyranoside,
the 3-O-(2-methyl-1-oxopropyl)-β-D-fructofuranosyl(1→2)-3,6-bis(2-methylpropanoate)-2-decanoate-α-D-glucopyranoside,
the 3-O-(2-methyl-1-oxopropyl)-β-D-fructofuranosyl(1→2)-3-(3-methylbutanoate)-2-decanoate-α-D-glucopyranoside.
the 3-O-(2-methyl-1-oxopropyl)-β-D-fructofuranosyl(1→2)-3,4-bis(2-methylpropanoate)-2-nonanoate -α-D-glucopyranoside,
the 3-O-(2-methyl-1-oxopropyl)-β-D-fructofuranosyl(1→2)-3,4-bis(2-methylpropanoate)-2-octanoate-α-D-glucopyranoside,
alone or in admixture with other active ingredients, which is intended to improve the appearance of the skin, mucous membranes or skin appendages, prevent and/or fight against the dryness of skin and mucous membranes, prevent and/or fight against the cutaneous signs of ageing and/or photo ageing.

7. Process for preparing a mixture of sucrose esters obtained from a plant extract according to claim 1, 2 or 6, comprising the following steps:
- drying the part of the plant,
- grinding,
- extraction with supercritical carbon dioxide with or without a co-solvent such as an alcohol or a plant oil or else an ester,
- desolvation if required, then
- purification, if required, according to one or more suitable methods.
